(19) Europäisches Patentamt
European Patent Office
Office européen des brevets

(11) Veröffentlichungsnummer: **0 248 861 B1**

(12) **EUROPÄISCHE PATENTSCHRIFT**

(45) Veröffentlichungstag der Patentschrift: **16.08.89**

(51) Int. Cl.⁴: **C 07 C 43/18**, C 07 C 121/46, C 07 C 13/72, C 07 C 23/18, C 07 C 43/21, C 07 C 49/385, C 07 C 49/587, C 09 K 19/32

(21) Anmeldenummer: **87900111.3**

(22) Anmeldetag: **08.12.86**

(86) Internationale Anmeldenummer: **PCT/EP 86/00722**

(87) Internationale Veröffentlichungsnummer: **WO 87/03581 (18.06.87 Gazette 87/13)**

(54) **DISPIROTETRADECANE.**

(30) Priorität: **13.12.85 DE 3543997**

(43) Veröffentlichungstag der Anmeldung: **16.12.87 Patentblatt 87/51**

(45) Bekanntmachung des Hinweises auf die Patenterteilung: **16.08.89 Patentblatt 89/33**

(84) Benannte Vertragsstaaten: **CH DE FR GB LI**

(56) Entgegenhaltungen:
**JP-A-59 152 343**

**Zeitschrift für Naturforschung B, vol. 41b, No: 8,1968, Verlag der Zeitschrift für Naturforschung, (Tübingen, DE) W.Calaminus et al.: "Das Dispiro(5.1.5.1) tetradecan-Gerüst als Baustein für thermotrope Flüssigkristalle", see pages 1011-1014, see the whole document**

(73) Patentinhaber: **MERCK PATENT GESELLSCHAFT MIT BESCHRÄNKTER HAFTUNG, Frankfurter Strasse 250 Postfach 4119, D-6100 Darmstadt (DE)**

(72) Erfinder: **VÖGTLE, Fritz, In der Asbach 10, D-5305 Alfter (DE)**
Erfinder: **CALAMINUS, Wolfgang, Königstr. 8, D-5330 Königswinter 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung betrifft Dispirotetradecane der Formel:

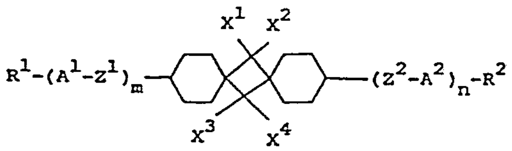

(I)

worin

$R^1$ und $R^2$ jeweils unabhängig voneinander Alkyl mit 1 bis 12 C-Atomen, worin auch eine oder mehrere nicht benachbarte $CH_2$-Gruppen durch $-O-$, $-CO-$, $-CO-O-$, $-O-CO$, $-O-COO-$ und/oder $-CH=CH-$ (trans) ersetzt sein können, einer der Reste $R^1$ und $R^2$ auch H, F, Cl, Br, J, CN, $NO_2$, NCS,

$A^1$ und $A^2$ jeweils unabhängig voneinander trans-1,4-Cyclohexylen, worin auch eine oder zwei nicht benachbarte $CH_2$-Gruppen durch $-O-$ und/oder $-S-$ ersetzt sein können oder 1,4-Phenylen, worin auch eine oder mehrere CH-Gruppen durch N ersetzt sein können, $A^1$ und $A^2$ gegebenenfalls auch lateral bzw. axial substituiert sein können durch F, Cl, CN, $CH_3$,

$Z^1$ und $Z^2$ jeweils unabhangig voneinander $-CO-O-$, $-O-CO$, $-CH_2CH_2-$, $-CH_2O-$, $-OCH_2-$ oder eine Einfachbindung,

m und n jeweils 0, 1 oder 2,

(m + n) 0, 1 oder 2,

$X^1$, $X^2$, $X^3$

und $X^4$ jeweils unbhängig voneinander H, F, Cl oder CN,

bedeuten, und eine oder beide der Gruppen $CX^1X^2$ und $CX^3X^4$ auch C=O bedeuten können, mit der Massgabe, dass (m + n) 1 oder 2 ist, wenn beide Gruppen $CX^1X^2$ und $CX^3X^4$ C=O bedeuten.

Der Einfachheit halber bedeuten im folgenden Cy eine 1,4-Cyclohexylengruppe, Dio eine 1,3-Dioxan-2,5-diylgruppe, Dit eine 1,3-Dithian-2,5-diylgruppe und Phe eine 1,4-Phenylengruppe.

Ähnliche Verbindungen sind z. B. aus DE-OS 34 26 035 und DE-OS 34 07 013 bekannt. Die dort angegebenen Verbindungen enthalten im Gegensatz zu den vorliegenden jedoch stets über eine Einfachbindung verknüpfte Cyclohexanringe, während die erfindungsgemässen Verbindungen stets die Dispirotetradecan-Struktur aufweisen.

In der JP-A-59 152 343 sind 3,11-disubstituierte Dispiro-[5.5.1]tetradecan- 7,14-dione mit Alkyl und/oder Alkoxy als Substituenten beschrieben. Diese Verbindungen eignen sich zur Herabsetzung der Schwellenspannung. Die erfindungsgemässen Verbindungen unterscheiden sich im Vergleich dazu durch zusätzliche Ringe im System oder dadurch, dass die Gruppen $CX^1X^2$ und $CX^3X^4$ verschieden von C=O sind.

Die Verbindungen der Formel I können wie ähnliche Verbindungen als Komponenten flüssigkristalliner Phasen verwendet werden, insbesondere für Displays, die auf dem Prinzip der verdrillten Zelle (TN-Displays), dem Guest-Host-Effekt, dem Effekt der Deformation aufgerichteter Phasen, dem Effekt der dynamischen Streuung oder dem SSFLC-Prinzip beruhen.

Der Erfindung lag die Aufgabe zugrunde, neue stabile flüssigkristalline oder mesogene Verbindungen aufzufinden, die als Komponenten flüssigkristalliner Phasen geeignet sind.

Es wurde nun gefunden, dass die Verbindungen der Formel I als Komponenten flüssigkristalliner Phasen vorzüglich geeignet sind. Insbesondere sind sie für Breitbereichsmischungen mit besonders niedriger optischer Anisotropie geeignet.

Mit der Bereitstellung der Verbindungen der Formel I wird ausserdem ganz allgemein die Palette der flüssigkristallinen Substanzen, die sich unter verschiedenen anwendungstechnischen Gesichtspunkten zur Herstellung flüssigkristalliner Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Phasen zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie oder andere Parameter eines solchen Dielektrikums zu optimieren. Die Verbindungen der Formel I eignen sich ferner als Zwischenprodukte zur Herstellung anderer Substanzen, die sich als Bestandteile flüssigkristalliner Phasen verwenden lassen.

Die Verbindungen der Formel I sind in reinem Zustand farblos und bilden flüssigkristalline Mesophasen in einem für die elektrooptische Verwendung günstig gelegenen Temperaturbereich. Chemisch, thermisch und gegen Licht sind sie sehr stabil.

Gegenstand der Erfindung sind somit die Verbindungen der Formel I sowie ein Verfahren zu ihrer Herstellung, dadurch gekennzeichnet, dass man entsprechend substituierte Cyclohexancarbonsäurehalogenide durch Dehydrohalogenierung in die Ketene überführt und diese zum Dispirotetradecandion-System dimerisiert,

oder dass man einer Verbindung, die sonst der Formel I entspricht, aber in 7,14-Position zwei C=O-Gruppen enthält, eine oder beide C=O-Gruppen mit einem reduzierenden Mittel behandelt,

oder dass man zur Herstellung von Estern der Formel I (worin $R^1$ und/oder $R^2$ eine Alkylgruppe bedeuten, worin eine oder mehrere $CH_2$-Gruppen durch $-O-CO$-Gruppen und/oder $-CO-O$-Gruppen ersetzt sind und/oder worin $Z^1$ und/oder $Z^2$ $-CO-O-$ oder $-O-CO-$ bedeuten) eine entsprechende Carbonsäure oder eines ihrer reaktionsfähigen Derivate mit einem entsprechenden Alkohol oder einem seiner reaktionsfähigen Derivate umsetzt,

oder dass man zur Herstellung von 1,3-Dioxanderivaten bzw. 1,3-Dithianderivaten der Formel I (worin $A^1$ und/oder $A^2$ 1,3-Dioxan-2,5-diyl bzw. 1,3-Dithian-2,5-diyl bedeutet) einen entsprechenden Aldehyd mit einem entsprechenden Diol bzw. Dithiol umsetzt,

oder dass man zur Herstellung von Ethern der Formel I (worin $R^1$ und/oder $R^2$ eine Alkylgruppe bedeutet, worin eine oder mehrere $CH_2$-Gruppen durch O-Atome ersetzt sind und/oder $Z^1$ und/oder $Z^2$ eine $-OCH_2-$ oder $-CH_2O$-Gruppe ist) eine entsprechende Hydroxyverbindung verethert,

oder dass man zur Herstellung von Nitrilen der Formel I (worin $X^1$, $X^2$, $X^3$ und/oder $X^4$ CN bedeutet) die entsprechenden Chlor- oder Bromverbindungen mit einem Cyanid umsetzt.

Weiterhin ist Gegenstand der Erfindung die Verwendung der Verbindungen der Formel I

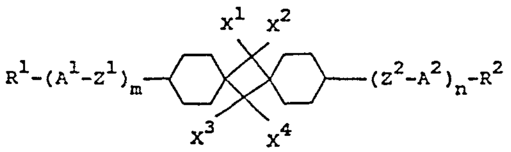 (I)

worin

$R^1$ und $R^2$ jeweils unabhängig voneinander Alkyl mit 1 bis 12 C-Atomen, worin auch eine oder mehrere nicht benachbarte $CH_2$-Gruppen durch $-O-$, $-CO-$, $-CO-O-$, $-O-CO-$, $-O-COO-$ und/oder $-CH=CH-$ (trans) ersetzt sein können, einer der Reste $R^1$ und $R^2$ auch H, F, Cl, Br, J, CN, $NO_2$, NCS,

$A^1$ und $A^2$ jeweils unabhangig voneinander trans-1,4-Cyclohexylen, worin auch eine oder zwei nicht benachbarte $CH_2$-Gruppen durch $-O-$ und/oder $-S-$ ersetzt sein können oder 1,4-Phenylen, worin auch eine oder mehrere CH-Gruppen durch N ersetzt sein können, $A^1$ und $A^2$ gegebenenfalls auch lateral bzw. axial substituiert sein können durch F, Cl, CN, $CH_3$,

$Z^1$ und $Z^2$ jeweils unabhängig voneinander $-CO-O-$, $-O-CO$, $-CH_2CH_2-$, $-CH_2O-$, $-OCH_2-$ oder eine Einfachbindung,

m und n jeweils 0, 1 oder 2,

(m + n) 0, 1 oder 2,

$X^1$, $X^2$, $X^3$

und $X^4$ jeweils unbhängig voneinander H, F, Cl oder CN,

bedeuten, und eine oder beide der Gruppen $CX^1X^2$ und $CX^3X^4$ auch C=O bedeuten können, mit der Massgabe, dass (m + n) 1 oder 2 ist, wenn beide Gruppen $CX^1X^2$ und $CX^3X^4$ C=O bedeuten, als Komponenten flüssigkristalliner Phasen mit einer besonders niedrigen optischen Anisotropie, vorzugsweise mit $\Delta n \leqq 0{,}09$, insbesondere $\Delta n \leqq 0{,}08$. Gegenstand der Erfindung sind auch flüssigkristalline Phasen mit mindestens zwei flüssigkristalline Komponenten, dadurch gekennzeichnet, dass mindestens eine Komponente der Phase eine Verbindung der Formel I ist.

Gegenstand der Erfindung sind ferner Flüssigkristallanzeigeelemente, insbesondere elektrooptische Anzeigeelemente, die derartige Phasen enthalten.

Vor- und nachstehend haben $R^1$, $R^2$, $A^1$, $A^2$, $Z^1$, $Z^2$, $X^1$, $X^2$, $X^3$, $X^4$, m und n die angegebene Bedeutung, sofern nicht ausdrücklich etwas anderes vermerkt ist.

Die Verbindungen der Formel I umfassen Verbindungen der Teilformeln Ia bis Id:

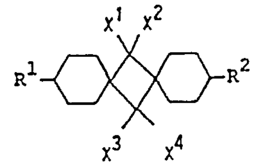 (Ia)

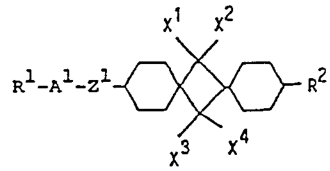 (Ib)

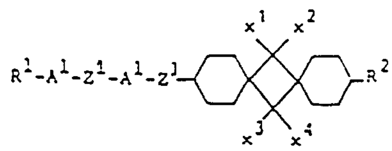 (Ic)

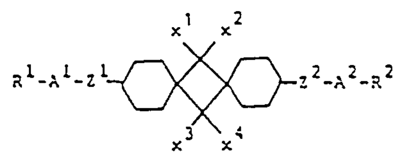 (Id)

Darunter sind diejenigen der Teilformel Ia und Id besonders bevorzugt.

Das Strukturelement

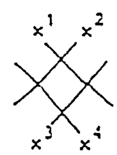

ist vorzugsweise

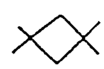 1

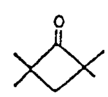 2

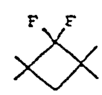 3

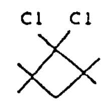 4

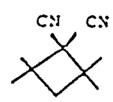 5

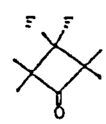 6

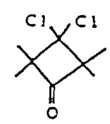 7

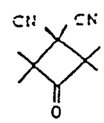 8

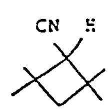 9

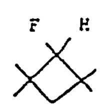 10

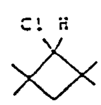 11

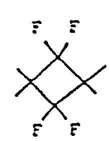 12

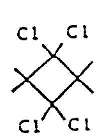 13

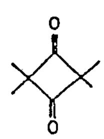 14

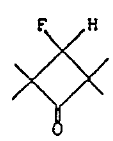 15

Besonders bevorzugt sind die Strukturelemente 1, 2, 3, 5, 7, 9, 10, 12, 14 und 15, insbesondere 1, 2 und 3.

Die bevorzugten Verbindungen der Teilformel Ia umfassen solche der Teilformeln Iaa bis Iag:

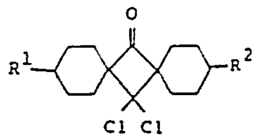 (Iaa)

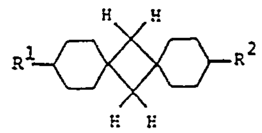 (Iab)

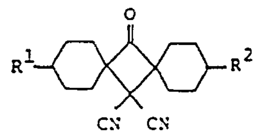 (Iac)

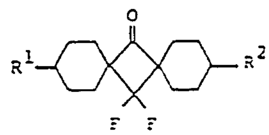 (Iad)

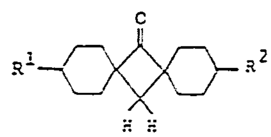 (Iae)

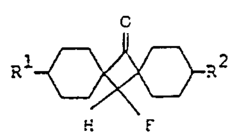 (Iaf)

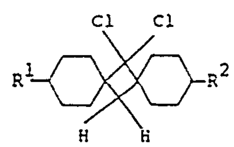 (Iag)

Darunter sind diejenigen der Teilformeln Iab, Iae und Iag besonders bevorzugt.

Die bevorzugten Verbindungen der Teilformel Ib umfassen solche der Teilformeln Iba bis Ibd:

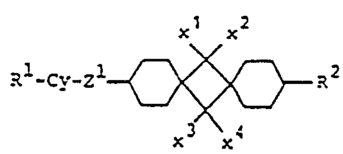 (Iba)

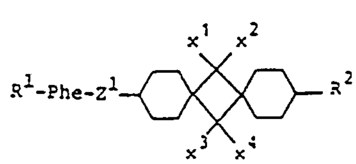 (Ibb)

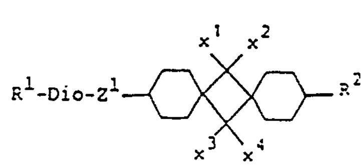 (Ibc)

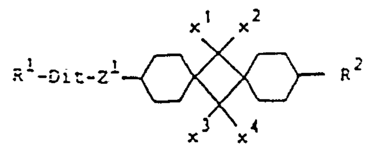 (Ibd)

In den Teilformeln Iba bis Ibd bedeutet $Z^1$ in erster Linie eine Einfachbindung, in zweiter Linie $-CO-O-$ oder $-O-CO-$, $X^1$-$X^4$ bedeuten vorzugsweise H, ferner auch Cl, F oder CN, eine oder beide der Gruppen $CX^1X^2$ und $CX^3X^4$ können auch C=O bedeuten.

Darunter sind diejenigen der Teilformel Iba besonders bevorzugt.

Die bevorzugten Verbindungen der Teilformel Ic umfassen solche der Teilformeln Ica bis Icd:

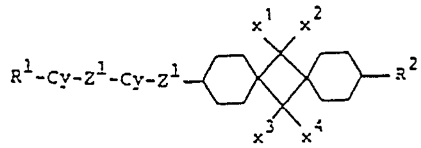 (Ica)

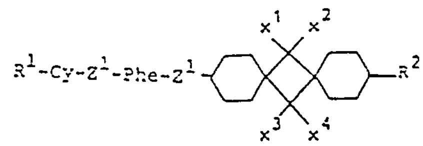 (Icb)

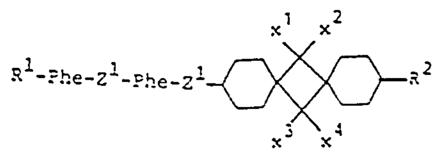 (Icc)

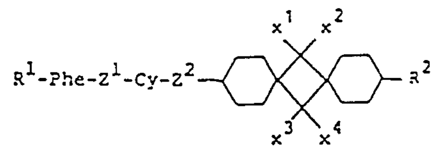 (Icd)

In Ica bis Icd bedeutet $Z^1$ vorzugsweise eine Einfachbindung, ferner auch $-CO-O-$ oder $-O-CO-$, $X^1$-$X^4$ bedeuten vorzugsweise H, ferner auch Cl, F oder CN, eine oder beide der Gruppen $CX^1X^2$ und $CX^3X^4$ können auch C=O bedeuten.

Darunter sind diejenigen der Teilformeln Ica und Icd besonders bevorzugt.

Die bevorzugten Verbindungen der Teilformel Id umfassen solche der Teilformeln Ida bis Ide:

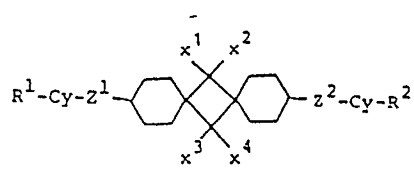 (Ida)

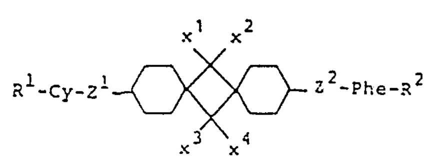 (Idb)

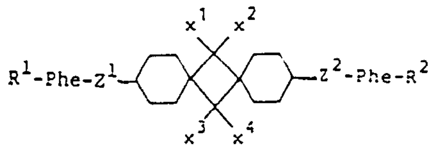

(Idc)

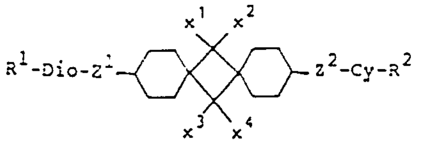

(Idd)

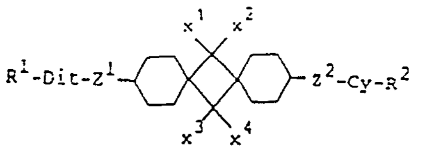

(Ide)

In den Teilformeln Ida bis Ide bedeuten $Z^1$ und/oder $Z^2$ in erster Linie Einfachbindungen, in zweiter Linie −CO−O oder −O−CO−; $X^1$-$X^4$ bedeuten vorzugsweise H, ferner auch Cl, CN oder F; eine oder beide der Gruppen $CX^1X^2$ und $CX^3X^4$ können auch C=O bedeuten.

Darunter sind diejenigen der Teilformel Ida besonders bevorzugt.

In den Verbindungen der vor- und nachstehenden Formeln bedeuten $R^1$ und $R^2$ vorzugsweise Alkyl, −O-Alkyl, −OCO-Alkyl oder −COO-Alkyl, insbesondere Alkyl, falls mit Phe verbunden auch CN, F, NCS, $NO_2$.

$A^1$ und $A^2$ sind bevorzugt Cy oder Phe.

$Z^1$ und $Z^2$ sind bevorzugt Einfachbindungen, ferner bevorzugt auch −CO−O−, −O−CO, $-CH_2O-$, $-OCH_2-$ oder $CH_2CH_2$-Gruppen.

$X^1$-$X^4$ bedeuten bevorzugt H, ferner F, Cl oder CN.

Ferner können eine oder beide der Gruppen $CX^1X^2$ und $CX^3X^4$ auch eine C=O-Gruppe sein.

Falls $CX^1X^2$ und $CX^3X^4$ C=O bedeuten, ist (m + n) 1 oder 2.

m und n sind vorzugsweise 0 oder 1 im Rahmen der in den Patentansprüchen gegebenen Definitionen.

Die Alkylreste in den Gruppen $R^1$ und/oder $R^2$ können geradkettig oder verzweigt sein. Vorzugsweise sind sie geradkettig, haben 1, 2, 3, 4, 5, 6, 7, 8, 9 oder 10 C-Atome und bedeuten demnach bevorzugt Methyl, Ethyl, Propyl, Butyl, Pentyl, Hexyl, Heptyl, Octyl, Nonyl oder Decyl, ferner Undecyl oder Dodecyl.

Falls $R^1$ und/oder $R^2$ Alkylreste bedeuten, in denen bevorzugt eine («Alkoxy» bzw. «Oxaalkyl») oder zwei («Alkoxyalkoxy» bzw. «Dioxaalkyl») $CH_2$-Gruppen durch O-Atome ersetzt sind, so können sie geradkettig oder verzweigt sein. Vorzugsweise sind sie geradkettig, haben 2, 3, 4, 5, 6 oder 7 C-Atome und bedeuten demnach bevorzugt Ethoxy, Propoxy, Butoxy, Pentoxy, Hexoxy, Heptoxy, 2-Oxapropyl (= Methoxymethyl), 2- (= Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, ferner Methoxy, Octoxy, Nonoxy, Decoxy, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl, 1,3-Dioxabutyl (= Methoxymethoxy), 1,3-, 1,4- oder 2,4-Dioxapentyl, 1,3-, 1,4-, 1,5-, 2,4-, oder 3,5-Dioxahexyl, 1,3-, 1,4-, 1,5-, 1,6-, 2,4-, 2,5-, 2,6-, 3,5-, 3,6- oder 4,6-Dioxaheptyl.

Verbindungen der Formel I sowie der vor- und nachstehenden Teilformeln mit verzweigten Flügelgruppen $R^1$ bzw. $R^2$ können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Solche Verbindungen eignen sich ferner als Komponenten ferroelektrischer flüssigkristalliner Phasen. Verzweigte Gruppen dieser Art enthalten in der Regel nicht mehr als eine Kettenverzweigung. Bevorzugte verzweigte Reste $R^1$ sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, 2-Octyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy, 2-Oxa-3-methylbutyl, 3-Oxa-4-methylpentyl, 2-Octyloxy.

Die Verbindungen der Formel I werden nach an sich bekannten Methoden hergestellt, wie sie in der Literatur (z. B. in den Standardwerken wie Houben Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht erwähnten Varianten Gebrauch machen.

Der Fachmann kann durch Routinemethoden entsprechende Synthesemethoden aus dem Stand der Technik entnehmen (z. B. JP 59-152343 betreffend die Dispiro-(5.1.5.1)-tetradecan-7,14-dione; DE-OS 23 44 732, 24 540 088, 24 29 093, 25 02 304, 26 36 684, 27 01 591 und 27 52 975 betreffend Verbindungen mit 1,4-Cyclohexylen- und 1,4-Phenylen-Gruppen; DE-OS 29 44 905 und 32 27 916 betreffend Verbindungen mit 1,3-Dioxan-2,5-diyl-Gruppen; und z. B. DE-OS 32 01 721 betreffend Verbindungen mit $-CH_2CH_2$-Brückengliedern.

Die Ausgangsstoffe können gewünschtenfalls auch in situ gebildet werden, derart, dass man sie aus dem Reaktionsgemisch nicht isoliert, sondern sofort weiter zu den Verbindungen der Formel I umsetzt.

So können die Verbindungen der Formel I hergestellt werden, indem man entsprechend substituierte Cyclohexancarbonsäurehalogenide in einem inerten Lösungsmittel, wie z. B. Benzol, Toluol, mit Hilfe einer Base unter Erhitzen dehydrohalogeniert und das entstandene Keten zum Dispirotetradecandion dimerisiert.

Ferner können die Verbindungen der Formel I hergestellt werden, indem man in entsprechenden 3,11-disubstituierten Dispirotetradecan-7,14-dionen, herstellbar z. B. durch Ketendimerisierung, eine oder beide Ketogruppen mit einem reduzierenden Mittel behandelt. Zur Einführung von

Halogensubstituenten wird das Dispirotetradecandion vorzugsweise in einem inerten Lösungsmittel, wie z. B. Tetrachlorkohlenstoff, Chloroform, Ether, mit einem anorganischen Säurehalogenid, z. B. Phosphorpentachlorid, Phosphorpentabromid, bei Temperaturen von 0-100°, vorzugsweise bei Raumtemperatur umgesetzt.

Verbindungen der Formel I können auch hergestellt werden, indem man eine Verbindung, die sonst der Formel I entspricht, aber an Stelle von H-Atomen eine oder mehrere reduzierbare Gruppen und/oder C-C-Bindungen enthält, reduziert.

Als reduzierbare Gruppen kommen vorzugsweise Carbonylgruppen in Betracht, insbesondere Ketogruppen, ferner z. B. freie oder veresterte Hydroxygruppen oder aromatisch gebundene Halogenatome. Bevorzugte Ausgangsstoffe für die Reduktion entsprechen der Formel I, können aber an Stelle eines Cyclohexanrings einen Cyclohexanring oder Cyclohexanonring und/oder an Stelle einer $-CH_2CH_2$-Gruppe eine $-CH=CH$-Gruppe und/oder an Stelle einer $-CH_2$-Gruppe eine $-CO$-Gruppe und/oder an Stelle eines H-Atomes eine freie oder eine funktionell (z. B. in Form ihres p-Toluolssulfonats) abgewandelte OH-Gruppe enthalten. Die Carbonylgruppen der Dispiroverbindung in 7- und 14-Position können ebenfalls durch übliche Reduktionsverfahren reduziert werden.

Die Reduktion kann z. B. erfolgen durch katalytische Hydrierung bei Temperaturen zwischen etwa 0° und etwa 200° sowie Drucken zwischen etwa 1 und 200 bar in einem inerten Lösungsmittel, z. B. einem Alkohol wie Methanol, Ethanol oder Isopropranol, einem Ether wie Tetrahydrofuran (THF) oder Dioxan, einem Ester wie Ethylacetat, einer Carbonsäure wie Essigsäure oder einem Kohlenwasserstoff wie Cyclohexan. Als Katalysatoren eignen sich zweckmässig Edelmetalle wie Pt oder Pd, die in Form von Oxiden (z. B. $PtO_2$, PdO), auf einem Träger (z. B. Pd auf Kohle, Calciumcarbonat oder Strontiumcarbonat) oder in feinverteilter Form eingesetzt werden können.

Ketone können auch nach den Methoden von Clemmensen (mit Zink, amalgamiertem Zink oder Zinn und Salzsäure, zweckmässig in wässerigalkoholischer Lösung oder in heterogener Phase mit Wasser/Toluol bei Temperaturen zwischen etwa 80 und 120° C) oder Wolff-Kishner (mit Hydrazin, zweckmässig in Gegenwart von Alkali wie KOH oder NaOH in einem hochsiedenden Lösungsmittel wie Diethylenglykol oder Triethylenglykol bei Temperaturen zwischen etwa 100 und 200° C) zu den entsprechenden Verbindungen der Formel I, die Alkylgruppen und/oder $-CH_2CH_2$-Brücken enthalten, reduziert werden. Bei der Empfindlichkeit des Vierring-Diketons können diese Methoden in bestimmten Fällen (je nach Substitution) nicht zum Erfolg führen. In solchen Fällen müssen schonendere Verfahren angewandt werden (Reduktion der Dihalogenide mit Natrium oder der Thioketone oder -ketale mit Raney-Nickel).

Weiterhin sind Reduktionen mit komplexen Hydriden möglich. Beispielsweise können Arylsulfonyloxygruppen mit $LiAlH_4$ reduktiv entfernt werden, insbesondere p-Toluolsulfonyloxymethylgruppen zu Methylgruppen reduziert werden, zweckmässig in einem inerten Lösungsmittel wie Diethylether oder THF bei Temperaturen zwischen etwa 0 und 100° C Doppelbindungen können (auch in Gegenwart von CN-Gruppen!) mit $NaBH_4$ oder Tributylzinnhydrid in Methanol hydriert werden.

Ester der Formel I ($R^1$ und/oder $R^2$ = Alkyl, worin eine oder mehrere $CH_2$-Gruppen durch $-O-CO-$ und/oder $-CO-O$-Gruppen ersetzt sind und/oder $Z^1$ und/oder $Z^2$ = $-CO-O-$ oder $-O-CO-$) können auch durch Veresterung entsprechender Carbonsäuren (oder ihren reaktionsfähigen Derivaten) mit Alkoholen bzw. Phenolen (oder ihrer reaktionsfähigen Derivaten) erhalten werden.

Als reaktionsfähige Derivate der genannten Carbonsäuren eignen sich insbesondere die Säurehalogenide, vor allem die Chloride und Bromide, ferner die Anhydride, Azide oder Ester, insbesondere Alkylester mit 1-4 C-Atomen in der Alkylgruppe.

Als reaktionsfähige Derivate der genannten Alkohole bzw. Phenole kommen insbesondere die entsprechenden Metallalkoholate bzw. Phenolate, vorzugsweise eines Alkalimetalls wie Na oder K, in Betracht.

Die Veresterung wird vorteilhaft in Gegenwart eines inerten Lösungsmittels durchgeführt. Gut geeignet sind insbesondere Ether wie Diethylether, Di-n-butylether, THF, Dioxan oder Anisol, Ketone wie Aceton, Butanon oder Cyclohexanon, Amide wie DMF oder Phosphorsäurehexamethyltriamid, Kohlenwasserstoffe wie Benzol, Toluol oder Xylol, Halogenkohlenwasserstoffe wie Tetrachlorkohlenstoff oder Tetrachlorethylen und Sulfoxide wie Dimethylsulfoxid oder Sulfolan. Mit Wasser nicht mischbare Lösungsmittel können gleichzeitig vorteilhaft zum azeotropen Abdestillieren des bei der Veresterung gebildeten Wassers verwendet werden. Gelegentlich kann auch ein Überschuss einer organischen Base, z. B. Pyridin, Chinolin oder Triethylamin als Lösungsmittel für die Veresterung angewandt werden. Die Veresterung kann auch in Abwesenheit eines Lösungsmittels, z. B. durch einfaches Erhitzen der Komponenten in Gegenwart von Natriumacetat, durchgeführt werden. Die Reaktionstemperatur liegt gewöhnlich zwischen −50° und +250°, vorzugsweise zwischen −20° und +80°. Bei diesen Temperaturen sind die Veresterungsreaktionen in der Regel nach 15 Minuten bis 48 Stunden beendet.

Im einzelnen hängen die Reaktionsbedingungen für die Veresterung weitgehend von der Natur der verwendeten Ausgangsstoffe ab. So wird eine freie Carbonsäure mit einem freien Alkohol oder Phenol in der Regel in Gegenwart einer starken Säure, beispielsweise einer Mineralsäure wie Salzsäure oder Schwefelsäure, umgesetzt. Eine bevorzugte Reaktionsweise ist die Umsetzung eines Säureanhydrids oder insbesondere eines

Säurechlorids mit einem Alkohol, vorzugsweise in einem basischen Milieu, wobei als Basen insbesondere Alkalimetallhydroxide wie Natrium- oder Kaliumhydroxid, Alkalimetallcarbonate bzw. -hydrogencarbonate wie Natriumcarbonat, Natriumhydrogencarbonat, Kaliumcarbonat oder Kaliumhydrogencarbonat, Alkalimetallacetate wie Natrium- oder Kaliumacetat, Erdalkalimetallhydroxide wie Calciumhydroxid oder organische Basen wie Triethylamin, Pyridin, Lutidin, Killidin oder Chinolin von Bedeutung sind. Eine weitere bevorzugte Ausführungsform der Veresterung besteht darin, dass man den Alkohol bzw. das Phenol zunächst in das Natrium- oder Kaliumalkoholat bzw. -phenolat überführt, z. B. durch Behandlung mit ethanolischer Natron- oder Kalilauge, dieses isoliert und zusammen mit Natriumhydrogencarbonat oder Kaliumcarbonat unter Rühren in Aceton oder Diethylether suspendiert und diese Suspension mit einer Lösung des Säurechlorids oder Anhydrids in Diethylether, Aceton oder DMF versetzt, zweckmässig bei Temperaturen zwischen etwa −25° und +20°.

Dioxanderivate bzw. Dithianderivate der Formel I (worin eine der Gruppen $A^1$ und/oder $A^2$ eine 1,3-Dioxan-2,5-diyl-Gruppe bzw. eine 1,3-Dithian-2,5-diyl-Gruppe bedeutet) werden zweckmässig durch Reaktion eines entsprechenden Aldehyds mit einem entsprechenden 1,3-Diol bzw. einem entsprechenden 1,3-Dithiol (oder einem seiner reaktionsfähigen Derivate) hergestellt, vorzugsweise in Gegenwart eines inerten Lösungsmittels wie Benzol oder Toluol und/oder eines Katalysators z. B. einer starken Säure wie Schwefelsäure, Benzol- oder p-Toluolsulfonsäure, bei Temperaturen zwischen etwa 20° und etwa 150°, vorzugsweise zwischen 80° und 120°. Als reaktionsfähige Derivate der Ausgangstoffe eignen sich in erster Linie Acetale.

Die genannten Aldehyde, 1,3-Diol und 1,3-Dithiole sowie ihre reaktionsfähigen Derivate sind zum Teil bekannt, zum Teil können sie ohne Schwierigkeiten nach Standardverfahren der organischen Chemie aus literaturbekannten Verbindungen hergestellt werden. Beispielsweise sind die Aldehyde durch Oxydation entsprechender Alkohole oder durch Reduktion entsprechender Carbonsäuren oder ihrer Derivate, die Diole durch Reduktion entsprechender Diester, sowie die Dithiole durch Umsetzung entsprechender Dihalogenide mit NaSH erhältlich.

Ether der Formel I (worin $R^1$ und/oder $R^2$ eine Alkylgruppe bedeutet, worin eine oder mehrere $CH_2$-Gruppen durch O-Atome ersetzt sind, und/oder worin $Z^1$ und/oder $Z^2$ eine $-OCH_2-$ oder eine $-CH_2O$-Gruppe ist) sind durch Veretherung entsprechender Hydroxyverbindungen, vorzugsweise entsprechender Phenole, erhältlich, wobei die Hydroxyverbindung zweckmässig zunächst in ein entsprechendes Metallderivat, z. B. durch Behandeln mit NaH, $NaNH_2$, NaOH, KOH, $Na_2CO_3$ oder $K_2CO_3$ in das entsprechende Alkalimetallalkoholat oder Alkalimetallphenolat übergeführt wird. Dieses kann dann mit dem entsprechenden Alkylhalogenid, -sulfonat oder Diakylsulfonat umgesetzt werden, zweckmässig in einem inerten Lösungsmittel wie Aceton, 1,2-Dimethoxyethan, DMF oder Dimethylsulfoxid oder auch einem Überschuss an wässeriger oder wässerig-alkoholischer NaOH oder KOH bei Temperaturen zwischen etwa 20° und 100°.

Zur Herstellung von Nitrilen der Formel I (worin $X^1$ und/oder $X^2$ und/oder $X^3$ und/oder $X^4$ CN bedeutet) können auch entsprechende Chlor- oder Bromverbindungen der Formel I mit einem Cyanid umgesetzt werden, zweckmässig mit einem Metallcyanid wie NaCN, KCN oder $Cu_2(CN)_2$, z. B. in Gegenwart von Pyridin in einem inerten Lösungsmittel wie DMF oder N-Methylpyrrolidon bei Temperaturen zwischen 20° und 200°.

Die erfindungsgemässen flüssigkristallinen Phasen bestehen aus 2 bis 15, vorzugsweise 3 bis 12 Komponenten, darunter mindestens einer Verbindung der Formel I. Die anderen Bestandteile werden vorzugsweise ausgewählt aus den nematischen oder nematogenen Substanzen, insbesondere den bekannten Substanzen, aus den Klassen der Azoxybenzole, Benzylidenaniline, Biphenyle, Terphenyle, Phenyl- oder Cyclohexylbenzoate, Cyclohexancarbonsäurephenyl- oder cyclohexylester, Phenylcyclohexane, Cyclohexylbiphenyle, Cyclohexylcyclohexane, Cyclohexylnaphthaline, 1,4-Bis-cyclohexylbenzole, 4,4'-Bis-cyclohexylbiphenyle, Phenyl- oder Cyclohexylpyrimidine, Phenyl- oder Cyclohexyldioxane, Phenyl- oder Cyclohexyl-1,3-dithiane, 1-Phenyl-2-cyclohexylethane, gegebenenfalls halogenierten Stilbene, Benzylphenylether, Tolane und substituierten Zimtsäuren.

Die wichtigsten als Bestandteile derartiger flüssigkristalliner Phasen in Frage kommenden Verbindungen lassen sich durch die Formel II charakterisieren,

$$R'-L-G-E-R'' \qquad (II)$$

worin L und E je ein carbo- oder heterocyclisches Ringsystem aus der aus 1,4-disubstituierten Benzol und Cyclohexanringen, 4,4'-disubstituierten Biphenyl-, Phenylcyclohexan- und Cyclohexylcyclohexansystemen, 2,5-disubstituierten Pyrimidin- und 1,3-Dioxanringen, 2,6-disubstituiertem Naphthalin, Di- und Tetrahydronaphthalin, Chinazolin und Tetrahydrochinazolin gebildeten Gruppe,

| | | |
|---|---|---|
| G | $-CH=CH-$ | $-N(O)=N-$ |
| | $-CH=CY-$ | $-CH=N(O)-$ |
| | $-C\equiv C-$ | $-CH_2-CH_2-$ |
| | $-CO-O-$ | $-CH_2-O-$ |
| | $-CO-S-$ | $-CH_2-S-$ |
| | $-CH=N-$ | $-COO-Phe-COO-$ |

oder eine C-C-Einfachbindung, Y Halogen, vorzugsweise Chlor, oder CN, und R' und R'' Alkyl, Alkoxy, Alkanoyloxy oder Alkoxycarbonyloxy mit bis zu 18, vorzugsweise bis zu 8 Kohlenstoffatomen, oder einer dieser Reste auch CN, NC, $NO_2$, $CH_3$, F, Cl oder Br bedeuten.

Bei den meisten dieser Verbindungen sind R' und R'' voneinander verschieden, wobei einer

dieser Reste meist eine Alkyl- oder Alkoxygruppe ist. Aber auch andere Varianten der vorgesehenen Substituenten sind gebräuchlich. Viele solcher Substanzen oder auch Gemische davon sind im Handel erhältlich. Alle diese Substanzen sind nach literaturbekannten Methoden herstellbar.

Die erfindungsgemässen Phasen enthalten etwa 0,1 bis 99, vorzugsweise 10 bis 95%, einer oder mehrerer Verbindungen der Formel I.

Weiterhin bevorzugt sind erfindungsgemässe Dielektrika enthaltend 0,1 bis 40, vorzugsweise 0,5 bis 30%, einer oder mehrerer Verbindungen der Formel I.

Die Herstellung der erfindungsgemässen Dielektrika erfolgt in an sich üblicher Weise. In der Regel werden die Komponenten ineinander gelöst, zweckmässig bei erhöhter Temperatur.

Durch geeignete Zusätze können die flüssigkristallinen Dielektrika nach der Erfindung so modifiziert werden, dass sie in allen bisher bekannt gewordenen Arten von Flüssigkristallanzeigeelementen verwendet werden können.

Derartige Zusätze sind dem Fachmann bekannt und in der Literatur ausführlich beschrieben. Beispielsweise können Leitsalze, vorzugsweise Ethyl-dimethyl-dodecyl-ammonium-4-hexyloxybenzoat, Tetrabutylammonium-tetraphenylboranat oder Komplexsalze von Kronenethern [vgl. z. B. I. Haller et al., Mol.Cryst.Liq.Cryst. Band *24*, Seiten 249-258 (1973)] zur Verbesserung der Leitfähigkeit, dichroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden. Derartige Substanzen sind z. B. in den DE-OS 22 09 127, 22 40 854, 23 21 632, 23 38 281, 24 50 088, 26 37 430, 28 53 728 und 29 02 177 beschrieben.

Die folgenden Beispiele sollen die Erfindung erläutern, ohne sie zu begrenzen. Fp. = Schmelzpunkt, Kp. = Klärpunkt. Vor- und nachstehend bedeuten Prozentangaben Gewichtsprozent; alle Temperaturen sind in Grad Celsius angegeben. K bedeutet kristalliner Zustand, N nematischer und I isotroper Zustand. Die Zahlen zwischen K, N und I stellen die Übergangstemperaturen dar.

*Beispiel 1:*

Zu einem Gemisch aus 5,4 g trans-4-(trans-4-n-Propyl-cyclohexyl)cyclohexancarbonsäurechlorid und 50 ml Benzol werden 3,0 g Triethylamin in 100 ml Benzol unter Rühren zugetropft, und anschliessend wird 8-24 h unter Rückfluss gerührt. Nach Abtrennen des Triethylammoniumchlorids wird das Filtrat neutral gewaschen und die organische Phase aufgearbeitet. Man erhält nach Umkristallisation 3,11-Bis(trans-4-n-propylcyclohexyl)dispiro-[5.1.5.1]-tetradecan-7,14-dion, nach chromatographischer Isomerentrennung erhält man die reine trans-Dispiroverbindung mit Fp. 290°.

Analog werden hergestellt:

3,11-Bis(trans-4-methylcyclohexyl)dispiro-[5.1.5.1]-tetradecan-7,14-dion

3,11-Bis(trans-4-ethylcyclohexyl)dispiro-[5.1.5.1]-tetradecan-7,14-dion

3,11-Bis(trans-4-butylcyclohexyl)dispiro-[5.1.5.1]-tetradecan-7,14-dion

3,11-Bis(trans-4-pentylcyclohexyl)dispiro-[5.1.5.1]-tetradecan-7,14-dion

3,11-Bis(trans-4-hexylcyclohexyl)dispiro-[5.1.5.1]-tetradecan-7,14-dion

3,11-Bis(trans-4-heptylcyclohexyl)dispiro-[5.1.5.1]-tetradecan-7,14-dion

3,11-Bis(trans-4-octylcyclohexyl)dispiro-[5.1.5.1]-tetradecan-7,14-dion

3,11-Bis(trans-4-nonylcyclohexyl)dispiro-[5.1.5.1]-tetradecan-7,14-dion

3,11-Bis(trans-4-decylcyclohexyl)dispiro-[5.1.5.1]-tetradecan-7,14-dion

3,11-Bis(trans-4-methoxycyclohexyl)dispiro-[5.1.5.1]-tetradecan-7,14-dion

3,11-Bis(trans-4-ethoxycyclohexyl)dispiro-[5.1.5.1]-tetradecan-7,14-dion

3,11-Bis(trans-4-propoxycyclohexyl)dispiro-[5.1.5.1]-tetradecan-7,14-dion

3,11-Bis(trans-4-butoxycyclohexyl)dispiro-[5.1.5.1]-tetradecan-7,14-dion

3,11-Bis(trans-4-pentoxycyclohexyl)dispiro-[5.1.5.1]-tetradecan-7,14-dion

3,11-Bis(trans-4-hexoxycyclohexyl)dispiro-[5.1.5.1]-tetradecan-7,14-dion

3,11-Bis(trans-4-heptoxycyclohexyl)dispiro-[5.1.5.1]-tetradecan-7,14-dion

3,11-Bis(trans-4-octoxycycyclohexyl)dispiro-[5.1.5.1]-tetradecan-7,14-dion

3,11-Bis(trans-4-nonoxycyclohexyl)dispiro-[5.1.5.1]-tetradecan-7,14-dion

3,11-Bis(trans-4-decoxycyclohexyl)dispiro-[5.1.5.1]-tetradecan-7,14-dion

3,11-Bis(4-methylphenyl)dispiro-[5.1.5.1]-tetradecan-7,14-dion

3,11-Bis(4-ethylphenyl)dispiro-[5.1.5.1]-tetradecan-7,14-dion

3,11-Bis(4-propylphenyl)dispiro-[5.1.5.1]-tetradecan-7,14-dion

3,11-Bis(4-butylphenyl)dispiro-[5.1.5.1]-tetradecan-7,14-dion

3,11-Bis(4-pentylphenyl)dispiro-[5.1.5.1]-tetradecan-7,14-dion

3,11-Bis(4-hexylphenyl)dispiro-[5.1.5.1]-tetradecan-7,14-dion

3,11-Bis(4-heptylphenyl)dispiro-[5.1.5.1]-tetradecan-7,14-dion

3,11-Bis(4-octylphenyl)dispiro-[5.1.5.1]-tetradecan-7,14-dion

3,11-Bis(4-nonylphenyl)dispiro-[5.1.5.1]-tetradecan-7,14-dion

3,11-Bis(4-decylphenyl)dispiro-[5.1.5.1]-tetradecan-7,14-dion

3,11-Bis(4-methoxyphenyl)dispiro-[5.1.5.1]-tetradecan-7,14-dion

3,11-Bis(4-ethoxyphenyl)dispiro-[5.1.5.1]-tetradecan-7,14-dion

3,11-Bis(4-propoxyphenyl)dispiro-[5.1.5.1]-tetradecan-7,14-dion

3,11-Bis(4-butoxyphenyl)dispiro-[5.1.5.1]-tetradecan-7,14-dion

3,11-Bis(4-pentoxyphenyl)dispiro-[5.1.5.1]-

tetradecan-7,14-dion
3,11-Bis(4-hexoxyphenyl)dispiro-[5.1.5.1]-tetradecan-7,14-dion
3,11-Bis(4-heptoxyphenyl)dispiro-[5.1.5.1]-tetradecan-7,14-dion
3,11-Bis(4-octoxyphenyl)dispiro-[5.1.5.1]-tetradecan-7,14-dion
3,11-Bis(4-nonoxyphenyl)dispiro-[5.1.5.1]-tetradecan-7,14-dion
3,11-Bis(4-decoxyphenyl)dispiro-[5.1.5.1]-tetradecan-7,14-dion
3-(trans-4-Methylcyclohexyl)-11-(trans-4-ethylcyclohexyl)-dispiro-[5.1.5.1]-tetradecan-7,14-dion
3-(trans-4-Ethylcyclohexyl)-11-(trans-4-pentylcyclohexyl)-dispiro-[5.1.5.1]-tetradecan-7,14-dion
3-(trans-4-Propylcyclohexyl)-11-(trans-4-ethylcyclohexyl)-dispiro-[5.1.5.1]-tetradecan-7,14-dion
3-(trans-4-Butylcyclohexyl)-11-(trans-4-ethylcyclohexyl)-dispiro-[5.1.5.1]-tetradecan-7,14-dion
3-(trans-4-Hexylcyclohexyl)-11-(trans-4-ethylcyclohexyl)-dispiro-[5.1.5.1]-tetradecan-7,14-dion
3-(trans-4-Ethylcyclohexyl)-11-(trans-4-heptylcyclohexyl)-dispiro-[5.1.5.1]-tetradecan-7,14-dion
3-(trans-4-Octylcyclohexyl)-11-(trans-4-propylcyclohexyl)-dispiro-[5.1.5.1]-tetradecan-7,14-dion
3-(trans-4-Butylcyclohexyl)-11-(trans-4-nonylcyclohexyl)-dispiro-[5.1.5.1]-tetradecan-7,14-dion
3-(trans-4-Decylcyclohexyl)-11-(trans-4-hexylcyclohexyl)-dispiro-[5.1.5.1]-tetradecan-7,14-dion
3-(trans-4-Propylcyclohexyl)-11-(trans-4-pentylcyclohexyl)-dispiro-[5.1.5.1]-tetradecan-7,14-dion
3-(trans-4-Pentylcyclohexyl)-11-(trans-4-heptylcyclohexyl)-dispiro-[5.1.5.1]-tetradecan-7,14-dion
3-(trans-4-Propylcyclohexyl)-11-trans-pentyldispiro-[5.1.5.1]-tetradecan-7,14-dion
3-(trans-4-Propylcyclohexyl)-11-trans-propyldispiro-[5.1.5.1]-tetradecan-7,14-dion
3-(trans-4-Propylcyclohexyl)-11-trans-ethyldispiro-[5.1.5.1]-tetradecan-7,14-dion
3-(trans-4-Propylcyclohexyl)-11-trans-butyldispiro-[5.1.5.1]-tetradecan-7,14-dion
3-(trans-4-Butylcyclohexyl)-11-trans-butyldispiro-[5.1.5.1]-tetradecan-7,14-dion
3-(trans-4-Propylcyclohexyl)-11-trans-hexyldispiro-[5.1.5.1]-tetradecan-7,14-dion
3-(trans-4-Pentylcyclohexyl)-11-trans-pentyldispiro-[5.1.5.1]-tetradecan-7,14-dion
3-(trans-4-Ethylcyclohexyl)-11-trans-pentyldispiro-[5.1.5.1]-tetradecan-7,14-dion
3-(trans-4-Methylcyclohexyl)-11-trans-propyldispiro-[5.1.5.1]-tetradecan-7,14-dion
3-(trans-4-Pentylcyclohexyl)-11-trans-methyldispiro-[5.1.5.1]-tetradecan-7,14-dion
3-(trans-4-Heptylcyclohexyl)-11-trans-heptyldispiro-[5.1.5.1]-tetradecan-7,14-dion
3-[trans-4-(p-Propylcyclohexyl)cyclohexyl]-11-trans-pentyldispiro-[5.1.5.1]-tetradecan-7,14-dion
3-[trans-4-(p-Propylcyclohexyl)cyclohexyl]-11-trans-propyldispiro-[5.1.5.1]-tetradecan-7,14-dion
3-[trans-4-(p-Propylcyclohexyl)cyclohexyl]-11-trans-ethyldispiro-[5.1.5.1]-tetradecan-7,14-dion
3-[trans-4-(p-Pentylcyclohexyl)cyclohexyl]-11-trans-pentyldispiro-[5.1.5.1]-tetradecan-7,14-dion
3-[trans-4-(p-Pentylcyclohexyl)cyclohexyl]-11-trans-propyldispiro-[5.1.5.1]-tetradecan-7,14-dion
3-[trans-4-(p-Ethylcyclohexyl)cyclohexyl]-11-trans-butyldispiro-[5.1.5.1]-tetradecan-7,14-dion
3-[trans-4-(p-Methylcyclohexyl)cyclohexyl]-11-trans-propyldispiro-[5.1.5.1]-tetradecan-7,14-dion
3-[trans-4-(p-Pentylcyclohexyl)cyclohexyl]-11-trans-methyldispiro-[5.1.5.1]-tetradecan-7,14-dion
3-[trans-4-(p-Heptylcyclohexyl)cyclohexyl]-11-trans-heptyldispiro-[5.1.5.1]-tetradecan-7,14-dion

*Beispiel 2:*

1 mmol 3,11-Bis(trans-4-propylcyclohexyl)dispiro-[5.1.5.1]-tetradecan-7,14-dion (erhältlich nach Bsp. 1) werden mit 210 mg (1 mmol) Phosphorpentachlorid in 30 ml Tetrachlorkohlenstoff 48 Stunden bei Raumtemperatur gerührt. Man giesst auf Eis/Wasser und arbeitet die organische Phase auf. Nach chromatographischer Reinigung erhält man 3,11-Bis(trans-4-propylcyclohexyl)-14,14-dichlordispiro-[5.1.5.1]-tetradecan-7-on, Fp. 159°, Kp. 266,7-270,1°.

Analog werden hergestellt:

3,11-Bis(trans-4-methylcyclohexyl)14,14-dichlordispiro-[5.1.5.1]-tetradecan-7-on
3,11-Bis(trans-4-ethylcyclohexyl)14,14-dichlordispiro-[5.1.5.1]-tetradecan-7-on
3,11-Bis(trans-4-butylcyclohexyl)14,14-dichlordispiro-[5.1.5.1]-tetradecan-7-on
3,11-Bis(trans-4-pentylcyclohexyl)14,14-dichlordispiro-[5.1.5.1]-tetradecan-7-on
3,11-Bis(trans-4-hexylcyclohexyl)14,14-dichlordispiro-[5.1.5.1]-tetradecan-7-on
3,11-Bis(trans-4-heptylcyclohexyl)14,14-dichlordispiro-[5.1.5.1]-tetradecan-7-on
3,11-Bis(trans-4-octylcyclohexyl)14,14-dichlordispiro-[5.1.5.1]-tetradecan-7-on
3,11-Bis(trans-4-nonylcyclohexyl)14,14-dichlordispiro-[5.1.5.1]-tetradecan-7-on
3,11-Bis(trans-4-decylcyclohexyl)14,14-dichlordispiro-[5.1.5.1]-tetradecan-7-on
3,11-Bis(trans-4-methoxycyclohexyl)14,14-dichlordispiro-[5.1.5.1]-tetradecan-7-on
3,11-Bis(trans-4-ethoxycyclohexyl)14,14-dichlordispiro-[5.1.5.1]-tetradecan-7-on
3,11-Bis(trans-4-propoxycyclohexyl)14,14-di-

chlordispiro-[5.1.5.1]-tetradecan-7-on
3,11-Bis(trans-4-butoxycyclohexyl)14,14-dichlordispiro-[5.1.5.1]-tetradecan-7-on
3,11-Bis(trans-4-pentoxycyclohexyl)14,14-dichlordispiro-[5.1.5.1]-tetradecan-7-on
3,11-Bis(trans-4-hexoxycyclohexyl)14,14-dichlordispiro-[5.1.5.1]-tetradecan-7-on
3,11-Bis(trans-4-heptoxycyclohexyl)14,14-dichlordispiro-[5.1.5.1]-tetradecan-7-on
3,11-Bis(trans-4-octoxycyclohexyl)14,14-dichlordispiro-[5.1.5.1]-tetradecan-7-on
3,11-Bis(trans-4-nonoxycyclohexyl)14,14-dichlordispiro-[5.1.5.1]-tetradecan-7-on
3,11-Bis(trans-4-decoxycyclohexyl)14,14-dichlordispiro-[5.1.5.1]-tetradecan-7-on
3,11-Bis(4-methylphenyl)-14,14-dichlordispiro-[5.1.5.1]-tetradecan-7-on
3,11-Bis(4-ethylphenyl)-14,14-dichlordispiro-[5.1.5.1]-tetradecan-7-on
3,11-Bis(4-propylphenyl)-14,14-dichlordispiro-[5.1.5.1]-tetradecan-7-on
3,11-Bis(4-butylphenyl)-14,14-dichlordispiro-[5.1.5.1]-tetradecan-7-on
3,11-Bis(4-pentylphenyl)-14,14-dichlordispiro-[5.1.5.1]-tetradecan-7-on
3,11-Bis(4-hexylphenyl)-14,14-dichlordispiro-[5.1.5.1]-tetradecan-7-on
3,11-Bis(4-heptylphenyl)-14,14-dichlordispiro-[5.1.5.1]-tetradecan-7-on
3,11-Bis(4-octylphenyl)-14,14-dichlordispiro-[5.1.5.1]-tetradecan-7-on
3,11-Bis(4-nonylphenyl)-14,14-dichlordispiro-[5.1.5.1]-tetradecan-7-on
3,11-Bis(4-decylphenyl)-14,14-dichlordispiro-[5.1.5.1]-tetradecan-7-on
3,11-Bis(4-methoxyphenyl)-14,14-dichlordispiro-[5.1.5.1]-tetradecan-7-on
3,11-Bis(4-ethoxyphenyl)-14,14-dichlordispiro-[5.1.5.1]-tetradecan-7-on
3,11-Bis(4-propoxyphenyl)-14,14-dichlordispiro-[5.1.5.1]-tetradecan-7-on
3,11-Bis(4-butoxyphenyl)-14,14-dichlordispiro-[5.1.5.1]-tetradecan-7-on
3,11-Bis(4-pentoxyphenyl)-14,14-dichlordispiro-[5.1.5.1]-tetradecan-7-on
3,11-Bis(4-hexoxyphenyl)-14,14-dichlordispiro-[5.1.5.1]-tetradecan-7-on
3,11-Bis(4-heptoxyphenyl)-14,14-dichlordispiro-[5.1.5.1]-tetradecan-7-on
3,11-Bis(4-octoxyphenyl)-14,14-dichlordispiro-[5.1.5.1]-tetradecan-7-on
3,11-Bis(4-nonoxyphenyl)-14,14-dichlordispiro-[5.1.5.1]-tetradecan-7-on
3,11-Bis(4-decoxyphenyl)-14,14-dichlordispiro-[5.1.5.1]-tetradecan-7-on
3-(trans-4-Methylcyclohexyl)-11-(trans-4-ethyl-cyclo-hexyl)-14,14-dichlorodispiro-[5.1.5.1]-tetradecan-7-on
3-(trans-4-Ethylcyclohexyl)-11-(trans-4-ethyl-cyclo-hexyl)-14,14-dichlorodispiro-[5.1.5.1]-tetradecan-7-on
3-(trans-4-Propylcyclohexyl)-11-(trans-4-ethyl-cyclo-hexyl)-14,14-dichlorodispiro-[5.1.5.1]-tetradecan-7-on
3-(trans-4-Butylcyclohexyl)-11-(trans-4-ethyl-cyclo-hexyl)-14,14-dichlorodispiro-[5.1.5.1]-tetradecan-7-on
3-(trans-4-Hexylcyclohexyl)-11-(trans-4-ethyl-cyclo-hexyl)-14,14-dichlorodispiro-[5.1.5.1]-tetradecan-7-on
3-(trans-4-Ethylcyclohexyl)-11-(trans-4-ethyl-cyclo-hexyl)-14,14-dichlorodispiro-[5.1.5.1]-tetradecan-7-on
3-(trans-4-Octylcyclohexyl)-11-(trans-4-propyl-cyclohexyl)-14,14-dichlorodispiro-[5.1.5.1]-tetradecan-7-on
3-(trans-4-Butylcyclohexyl)-11-(trans-4-nonyl-cyclohexyl)-14,14-dichlorodispiro-[5.1.5.1]-tetradecan-7-on
3-(trans-4-Decylcyclohexyl)-11-(trans-4-hexyl-cyclohexyl)-14,14-dichlorodispiro-[5.1.5.1]-tetradecan-7-on

*Beispiel 3:*

Analog Beispiel 2 erhält man aus 3,11-trans-Dipentyl-dispiro-[5.1.5.1]-tetradecan-7,14-dion (erhältlich nach JP 59-152343) das 3,11-trans-Dipentyl-14,14-dichlor-dispiro-[5.1.5.1]-tetradecan-7-on, Fp. 290°, Kp. 21°.

Analog werden hergestellt:

3,11-trans-Dimethyl-14,14-dichlordispiro-[5.1.5.1]-tetradecan-7-on, K 67° I
3,11-trans-Diethyl-14,14-dichlordispiro-[5.1.5.1]-tetradecan-7-on
3,11-trans-Dipropyl-14,14-dichlordispiro-[5.1.5.1]-tetradecan-7-on, Fp. 46°, Kp. −40° (extr.)
3,11-trans-Dibutyl-14,14-dichlordispiro-[5.1.5.1]-tetradecan-7-on
3,11-trans-Dihexyl-14,14-dichlordispiro-[5.1.5.1]-tetradecan-7-on
3,11-trans-Diheptyl-14,14-dichlordispiro-[5.1.5.1]-tetradecan-7-on
3,11-trans-Dioctyl-14,14-dichlordispiro-[5.1.5.1]-tetradecan-7-on
3,11-trans-Dinonyl-14,14-dichlordispiro-[5.1.5.1]-tetradecan-7-on
3,11-trans-Didecyl-14,14-dichlordispiro-[5.1.5.1]-tetradecan-7-on
3,11-trans-Dimethoxy-14,14-dichlordispiro-[5.1.5.1]-tetradecan-7-on
3,11-trans-Diethoxy-14,14-dichlordispiro-[5.1.5.1]-tetradecan-7-on
3,11-trans-Dipropoxy-14,14-dichlordispiro-[5.1.5.1]-tetradecan-7-on
3,11-trans-Dibutoxy-14,14-dichlordispiro-[5.1.5.1]-tetradecan-7-on
3,11-trans-Dipentoxy-14,14-dichlordispiro-[5.1.5.1]-tetradecan-7-on
3,11-trans-Dihexoxy-14,14-dichlordispiro-[5.1.5.1]-tetradecan-7-on
3,11-trans-Diheptoxy-14,14-dichlordispiro-[5.1.5.1]-tetradecan-7-on
3,11-trans-Dioctoxy-14,14-dichlordispiro-[5.1.5.1]-tetradecan-7-on
3,11-trans-Dinonoxy-14,14-dichlordispiro-[5.1.5.1]-tetradecan-7-on
3,11-trans-Didecoxy-14,14-dichlordispiro-[5.1.5.1]-tetradecan-7-on
3-trans-Methyl-11-trans-propyl-14,14-dichlor-dispiro[5.1.5.1]-tetradecan-7-on
3-trans-Propyl-11-trans-pentyl-14,14-dichlor-dispiro[5.1.5.1]-tetradecan-7-on
3-trans-Butyl-11-trans-ethyl-14,14-dichlor-dispiro[5.1.5.1]-tetradecan-7-on
3-trans-Pentyl-11-trans-heptyl-14,14-dichlor-dispiro[5.1.5.1]-tetradecan-7-on
3-trans-Octyl-11-trans-propyl-14,14-dichlor-dispiro[5.1.5.1]-tetradecan-7-on
3-trans-Propyl-11-trans-ethyl-14,14-dichlor-dispiro[5.1.5.1]-tetradecan-7-on

*Beispiel 4:*

Ein Gemisch aus 1 mmol 3,11-Bis(trans-4-propylcyclohexyl)-dispiro-[5.1.5.1]-tetradecan-7,14-dithion (herstellbar durch Umsetzung des entspr. Diketons mit $P_4S_{10}$) und 25 ml Tetrahydrofuran wird bei Raumtemperatur zu einer gut gerührten Aufschlämmung von frischem Raney-Nickel in THF gegeben und 24 h bei Raumtemperatur gerührt. Die übliche Aufarbeitung liefert 3,11-Bis(trans-4-propylcyclohexyl-dispiro-[5.1.5.1]-tetradecan.

Analog werden hergestellt:

3,11-Bis-(trans-4-methylcyclohexyl)-dispiro-[5.1.5.1]-tetradecan
3,11-Bis-(trans-4-ethylcyclohexyl)-dispiro-[5.1.5.1]-tetradecan
3,11-Bis-(trans-4-butylcyclohexyl)-dispiro-[5.1.5.1]-tetradecan
3,11-Bis-(trans-4-pentylcyclohexyl)-dispiro-[5.1.5.1]-tetradecan
3,11-Bis-(trans-4-hexylcyclohexyl)-dispiro-[5.1.5.1]-tetradecan
3,11-Bis-(trans-4-heptylcyclohexyl)-dispiro-[5.1.5.1]-tetradecan
3,11-Bis-(trans-4-octylcyclohexyl)-dispiro-[5.1.5.1]-tetradecan
3,11-Bis-(trans-4-nonylcyclohexyl)-dispiro-[5.1.5.1]-tetradecan
3,11-Bis-(trans-4-decylcyclohexyl)-dispiro-[5.1.5.1]-tetradecan
3,11-Bis-(trans-4-methoxycyclohexyl)-dispiro-[5.1.5.1]-tetradecan
3,11-Bis-(trans-4-ethoxycyclohexyl)-dispiro-[5.1.5.1]-tetradecan
3,11-Bis-(trans-4-propoxycyclohexyl)-dispiro-[5.1.5.1]-tetradecan
3,11-Bis-(trans-4-butoxycyclohexyl)-dispiro-[5.1.5.1]-tetradecan
3,11-Bis-(trans-4-pentoxycyclohexyl)-dispiro-[5.1.5.1]-tetradecan
3,11-Bis-(trans-4-hexoxycyclohexyl)-dispiro-[5.1.5.1]-tetradecan
3,11-Bis-(trans-4-heptoxycyclohexyl)-dispiro-[5.1.5.1]-tetradecan
3,11-Bis-(trans-4-octoxycyclohexyl)-dispiro-[5.1.5.1]-tetradecan
3,11-Bis-(trans-4-nonoxycyclohexyl)-dispiro-[5.1.5.1]-tetradecan
3,11-Bis-(trans-4-decoxycyclohexyl)-dispiro-[5.1.5.1]-tetradecan
3-(trans-4-Methylcyclohexyl)-11-(trans-4-ethylcyclohexyl)-dispiro-[5.1.5.1]-tetradecan
3-(trans-4-Ethylcyclohexyl)-11-(trans-4-pentylcyclohexyl)-dispiro-[5.1.5.1]-tetradecan
3-(trans-4-Propylcyclohexyl)-11-(trans-4-pentylcyclohexyl)-dispiro-[5.1.5.1]-tetradecan
3-(trans-4-Butylcyclohexyl)-11-(trans-4-ethylcyclohexyl)-dispiro-[5.1.5.1]-tetradecan
3-(trans-4-Pentylcyclohexyl)-11-(trans-4-heptylcyclohexyl)-dispiro-[5.1.5.1]-tetradecan
3-(trans-4-Octylcyclohexyl)-11-(trans-4-propylcyclohexyl)-dispiro-[5.1.5.1]-tetradecan
3-(trans-4-Propylcyclohexyl)-11-(trans-4-ethylcyclohexyl)-dispiro-[5.1.5.1]-tetradecan

*Beispiel 5:*

Ein Gemisch aus 1,96 g 3,11-trans-Dipentyldispiro-[5.1.5.1]-tetradecan-7,14-dithion (erhältlich durch Umsetzung des entsprechenden Diketons mit $P_4S_{10}$) und 25 ml THF werden bei Raumtemperatur zu einer gut gerührten Aufschlämmung von frischem Raney-Nickel in THF gegeben und 24 h bei Raumtemperatur gerührt. Nach üblicher Aufarbeitung und Umkristallisation aus Ethanol erhält man 3,11-trans-Dipentyl-dispiro-[5.1.5.1]-tetradecan mit Fp. 51° und Kp. 102°.

Analog werden hergestellt:

3,11-trans-Dimethyl-dispiro-[5.1.5.1]-tetradecan
3,11-trans-Diethyl-dispiro-[5.1.5.1]-tetradecan
3,11-trans-Dipropyl-dispiro-[5.1.5.1]-tetradecan
3,11-trans-Dibutyl-dispiro-[5.1.5.1]-tetradecan
3,11-trans-Dihexyl-dispiro-[5.1.5.1]-tetradecan
3,11-trans-Diheptyl-dispiro-[5.1.5.1]-tetradecan
3,11-trans-Dioctyl-dispiro-[5.1.5.1]-tetradecan
3,11-trans-Dinonyl-dispiro-[5.1.5.1]-tetradecan
3,11-trans-Didecyl-dispiro-[5.1.5.1]-tetradecan
3,11-trans-Dimethoxy-dispiro-[5.1.5.1]-tetradecan
3,11-trans-Diethoxy-dispiro-[5.1.5.1]-tetradecan
3,11-trans-Dipropoxy-dispiro-[5.1.5.1]-tetradecan
3,11-trans-Dibutoxy-dispiro-[5.1.5.1]-tetradecan
3,11-trans-Dipentoxy-dispiro-[5.1.5.1]-tetradecan
3,11-trans-Dihexoxy-dispiro-[5.1.5.1]-tetradecan
3,11-trans-Diheptoxy-dispiro-[5.1.5.1]-tetradecan
3,11-trans-Dioctoxy-dispiro-[5.1.5.1]-tetradecan

3,11-trans-Dinonoxy-dispiro-[5.1.5.1]-tetradecan
3,11-trans-Didecoxy-dispiro-[5.1.5.1]-tetradecan
3,11-trans-Methyl-11-trans-propyl-dispiro-[5.1.5.1]-tetradecan
3,11-trans-Propyl-11-trans-pentyl-dispiro-[5.1.5.1]-tetradecan
3,11-trans-Butyl-11-trans-ethyl-dispiro-[5.1.5.1]-tetradecan
3,11-trans-Pentyl-11-trans-heptyl-dispiro-[5.1.5.1]-tetradecan
3,11-trans-Octyl-11-trans-propyl-dispiro-[5.1.5.1]-tetradecan
3,11-trans-Propyl-11-trans-ethyl-dispiro-[5.1.5.1]-tetradecan

*Beispiel 6:*

1 mmol 3-(trans-4-Propylcyclohexyl)-11-trans-pentyl-dispiro-[5.1.5.1]-tetradecan-7,14-dion (erhältlich nach Beispiel 1 aus trans-4-(p-Propylcyclohexyl)cyclohexancarbonsäurechlorid und trans-4-Pentylcyclohexancarbonsäurechlorid) werden mit 210 mg Phosphorpentachlorid in 30 ml Tetrachlorkohlenstoff 48 Stunden bei Raumtemperatur gerührt. Man giesst auf Eis/Wasser und arbeitet die organische Phase aus. Nach chromatographischer Reinigung erhält man 3-(trans-4-Propylcyclohexyl)-11-trans-pentyl-14,14-dichlordispiro-[5.1.5.1]-tetradecan-7-on.

Analog werden hergestellt:

3-(trans-4-Propylcyclohexyl)-11-trans-propyl-14,14-dichlordispiro-[5.1.5.1]-tetradecan-7-on
3-(trans-4-Propylcyclohexyl)-11-trans-ethyl-14,14-dichlordispiro-[5.1.5.1]-tetradecan-7-on
3-(trans-4-Propylcyclohexyl)-11-trans-butyl-14,14-dichlordispiro-[5.1.5.1]-tetradecan-7-on
3-(trans-4-Butylcyclohexyl)-11-trans-butyl-14,14-dichlordispiro-[5.1.5.1]-tetradecan-7-on
3-(trans-4-Propylcyclohexyl)-11-trans-hexyl-14,14-dichlordispiro-[5.1.5.1]-tetradecan-7-on
3-(trans-4-Pentylcyclohexyl)-11-trans-pentyl-14,14-dichlordispiro-[5.1.5.1]-tetradecan-7-on
3-(trans-4-Ethylcyclohexyl)-11-trans-pentyl-14,14-dichlordispiro-[5.1.5.1]-tetradecan-7-on
3-(trans-4-Methylcyclohexyl)-11-trans-propyl-14,14-dichlordispiro-[5.1.5.1]-tetradecan-7-on
3-(trans-4-Pentylcyclohexyl)-11-trans-methyl-14,14-dichlordispiro-[5.1.5.1]-tetradecan-7-on
3-(trans-4-Heptylcyclohexyl)-11-trans-heptyl-14,14-dichlordispiro-[5.1.5.1]-tetradecan-7-on

*Beispiel 7:*

Nach Beispiel 6 erhält man ausgehend von 3-[trans-4-(p-Propylcyclohexyl)cyclohexyl]-11-trans-pentyldispiro-[5.1.5.1]tetradecan-7,14-dion (darstellbar nach Beispiel 1 aus trans-4-Pentylcyclohexancarbonsäurechlorid und trans-4-(p-Propylcyclohexyl)cyclohexyl-cyclohexancarbonsäurechlorid) 3-[trans-4-(p-Propylcyclohexyl)cyclohexyl]-11-trans-pentyl-14,14-dichlordispiro-[5.1.5.1]-tetradecan-7-on.

Analog werden hergestellt:

3-[trans-4-(p-Propylcyclohexyl)cyclohexyl]-11-trans-propyl-14,14-dichlordispiro-[5.1.5.1]-tetradecan-7-on
3-[trans-4-(p-Propylcyclohexyl)cyclohexyl]-11-trans-ethyl-14,14-dichlordispiro-[5.1.5.1]-tetradecan-7-on
3-[trans-4-(p-Propylcyclohexyl)cyclohexyl]-11-trans-butyl-14,14-dichlordispiro-[5.1.5.1]-tetradecan-7-on
3-[trans-4-(p-Pentylcyclohexyl)cyclohexyl]-11-trans-pentyl-14,14-dichlordispiro-[5.1.5.1]-tetradecan-7-on
3-[trans-4-(p-Pentylcyclohexyl)cyclohexyl]-11-trans-propyl-14,14-dichlordispiro-[5.1.5.1]-tetradecan-7-on
3-[trans-4-(p-Ethylcyclohexyl)cyclohexyl]-11-trans-butyl-14,14-dichlordispiro-[5.1.5.1]-tetradecan-7-on
3-[trans-4-(p-Methylcyclohexyl)cyclohexyl]-11-trans-propyl-14,14-dichlordispiro-[5.1.5.1]-tetradecan-7-on
3-[trans-4-(p-Pentylcyclohexyl)cyclohexyl]-11-trans-methyl-14,14-dichlordispiro-[5.1.5.1]-tetradecan-7-on
3-[trans-4-(p-Heptylcyclohexyl)cyclohexyl]-11-trans-heptyl-14,14-dichlordispiro-[5.1.5.1]-tetradecan-7-on

*Beispiel 8:*

Analog Beispiel 4 erhält man aus 3-(trans-4-Propylcyclohexyl)-11-trans-pentyldispiro-[5.1.5.1]-tetradecan-7,14-dithion 3-(trans-4-Propylcyclohexyl)-11-trans-pentyl-dispiro-[5.1.5.1]-tetradecan.

Analog werden hergestellt:

3-(trans-4-Propylcyclohexyl)-11-trans-propyl-dispiro-[5.1.5.1]-tetradecan
3-(trans-4-Propylcyclohexyl)-11-trans-ethyl-dispiro-[5.1.5.1]-tetradecan
3-(trans-4-Propylcyclohexyl)-11-trans-butyl-dispiro-[5.1.5.1]-tetradecan
3-(trans-4-Butylcyclohexyl)-11-trans-butyl-dispiro-[5.1.5.1]-tetradecan
3-(trans-4-Propylcyclohexyl)-11-trans-hexyl-dispiro-[5.1.5.1]-tetradecan
3-(trans-4-Pentylcyclohexyl)-11-trans-pentyl-dispiro-[5.1.5.1]-tetradecan
3-(trans-4-Ethylcyclohexyl)-11-trans-pentyl-dispiro-[5.1.5.1]-tetradecan
3-(trans-4-Methylcyclohexyl)-11-trans-propyl-dispiro-[5.1.5.1]-tetradecan
3-(trans-4-Pentylcyclohexyl)-11-trans-methyl-dispiro-[5.1.5.1]-tetradecan
3-(trans-4-Heptylcyclohexyl)-11-trans-heptyl-dispiro-[5.1.5.1]-tetradecan

*Beispiel 9:*

Analog Beispiel 4 erhält man ausgehend von 3-[trans-4-(p-Propylcyclohexyl)cyclohexyl]-11-trans-pentyldispiro-[5.1.5.1]-tetradecan-7,14-dithion-3-[trans-4-(p-Propyl-cyclohexyl)cyclohexyl]-11-trans-pentyl-dispiro-[5.1.5.1]-tetra decan.

Analog werden hergestellt:

3-[trans-4-(p-Propylcyclohexyl)cyclohexyl]-11-trans-propyl-dispiro-[5.1.5.1]-tetradecan
3-[trans-4-(p-Propylcyclohexyl)cyclohexyl]-11-trans-ethyl-dispiro-[5.1.5.1]-tetradecan
3-[trans-4-(p-Propylcyclohexyl)cyclohexyl]-11-trans-butyl-dispiro-[5.1.5.1]-tetradecan
3-[trans-4-(p-Butylcyclohexyl)cyclohexyl]-11-trans-butyl-dispiro-[5.1.5.1]-tetradecan
3-[trans-4-(p-Propylcyclohexyl)cyclohexyl]-11-trans-hexyl-dispiro-[5.1.5.1]-tetradecan
3-[trans-4-(p-Pentylcyclohexyl)cyclohexyl]-11-trans-pentyl-dispiro-[5.1.5.1]-tetradecan
3-[trans-4-(p-Ethylcyclohexyl)cyclohexyl]-11-trans-pentyl-dispiro-[5.1.5.1]-tetradecan
3-[trans-4-(p-Methylcyclohexyl)cyclohexyl]-11-trans-propyl-dispiro-[5.1.5.1]-tetradecan
3-[trans-4-(p-Pentylcyclohexyl)cyclohexyl]-11-trans-methyl-dispiro-[5.1.5.1]-tetradecan
3-[trans-4-(p-Heptylcyclohexyl)cyclohexyl]-11-trans-heptyl-dispiro-[5.1.5.1]-tetradecan

*Beispiel 10:*

Unter Ausschluss von Luftfeuchtigkeit wird bei 10°C zu einer Lösung von 5,2 g Diethylaminoschwefeltrifluorid in 30 ml Dichlormethan eine Lösung von 10 g 3,11-trans-Dipentyl-dispiro-[5.1.5.1]-tetradecan-7-ol-14-on (herstellbar aus 3,11-trans-Dipentyl-[5.1.5.1]-tetradecan-7,14-dion durch Umsetzung mit Lithiumaluminiumhydrid) in 50 ml Dichlormethan zugegeben. Das Reaktionsgemisch wird innerhalb einer Stunde auf Raumtemperatur erwärmt und noch 1 Stunde stehengelassen. Danach wird auf Eis gegossen und mit Dichlormethan extrahiert. Nach Waschen der organischen Phasen, Abdampfen des Lösungsmittels und Umkristallisation des Rückstandes erhält man 3,11-trans-Dipentyl-7-fluor-dispiro-[5.1.5.1]-tetradecan-14-on mit Fp. 53° und Kp. 116°.

Analog werden hergestellt:

3,11-trans-Dimethyl-7-fluor-dispiro-[5.1.5.1]-tetradecan-14-on
3,11-trans-Diethyl-7-fluor-dispiro-[5.1.5.1]-tetradecan-14-on
3,11-trans-Dipropyl-7-fluor-dispiro-[5.1.5.1]-tetradecan-14-on
3,11-trans-Dibutyl-7-fluor-dispiro-[5.1.5.1]-tetradecan-14-on
3,11-trans-Dihexyl-7-fluor-dispiro-[5.1.5.1]-tetradecan-14-on
3,11-trans-Diheptyl-7-fluor-dispiro-[5.1.5.1]-tetradecan-14-on
3,11-trans-Dioctyl-7-fluor-dispiro-[5.1.5.1]-tetradecan-14-on
3,11-trans-Dinonyl-7-fluor-dispiro-[5.1.5.1]-tetradecan-14-on
3,11-trans-Didecyl-7-fluor-dispiro-[5.1.5.1]-tetradecan-14-on
3,11-trans-Dimethoxy-7-fluor-dispiro-[5.1.5.1]-tetradecan-14-on
3,11-trans-Diethoxy-7-fluor-dispiro-[5.1.5.1]-tetradecan-14-on
3,11-trans-Dipropoxy-7-fluor-dispiro-[5.1.5.1]-tetradecan-14-on
3,11-trans-Dibutoxy-7-fluor-dispiro-[5.1.5.1]-tetradecan-14-on
3,11-trans-Dipentoxy-7-fluor-dispiro-[5 1.5.1]-tetradecan-14-on
3,11-trans-Dihexoxy-7-fluor-dispiro-[5.1.5.1]-tetradecan-14-on
3,11-trans-Diheptoxy-7-fluor-dispiro-[5.1.5.1]-tetradecan-14-on
3,11-trans-Dioctoxy-7-fluor-dispiro-[5.1.5.1]-tetradecan-14-on
3,11-trans-Dinonoxy-7-fluor-dispiro-[5.1.5.1]-tetradecan-14-on
3,11-trans-Didecoxy-7-fluor-dispiro-[5.1.5.1]-tetradecan-14-on
3-trans-Methyl-11-trans-propyl-7-fluor-dispiro-[5.1.5.1]-tetradecan-14-on
3-trans-Propyl-11-trans-pentyl-7-fluor-dispiro-[5.1.5.1]-tetradecan-14-on
3-trans-Butyl-11-trans-ethyl-7-fluor-dispiro-[5.1.5.1]-tetradecan-14-on
3-trans-Pentyl-11-trans-heptyl-7-fluor-dispiro-[5.1.5.1]-tetradecan-14-on
3-trans-Octyl-11-trans-propyl-7-fluor-dispiro-[5.1.5.1]-tetradecan-14-on
3-trans-Propyl-11-trans-ethyl-7-fluor-dispiro-[5.1.5.1]-tetradecan-14-on
3,11-Bis(trans-4-propylcyclohexyl)-7-fluor-dispiro-[5.1.5.1]-tetradecan-14-on
3,11-Bis(trans-4-methylcyclohexyl)-7-fluor-dispiro-[5.1.5.1]-tetradecan-14-on
3,11-Bis(trans-4-ethylcyclohexyl)-7-fluor-dispiro-[5.1.5.1]-tetradecan-14-on
3,11-Bis(trans-4-butylcyclohexyl)-7-fluor-dispiro-[5.1.5.1]-tetradecan-14-on
3,11-Bis(trans-4-pentylcyclohexyl)-7-fluor-dispiro-[5.1.5.1]-tetradecan-14-on
3,11-Bis(trans-4-hexylcyclohexyl)-7-fluor-dispiro-[5.1.5.1]-tetradecan-14-on
3,11-Bis(trans-4-heptylcyclohexyl)-7-fluor-dispiro-[5.1.5.1]-tetradecan-14-on
3,11-Bis(trans-4-octylcyclohexyl)-7-fluor-dispiro-[5.1.5.1]-tetradecan-14-on
3,11-Bis(trans-4-nonylcyclohexyl)-7-fluor-dispiro-[5.1.5.1]-tetradecan-14-on
3,11-Bis(trans-4-decylcyclohexyl)-7-fluor-dispiro-[5.1.5.1]-tetradecan-14-on
3,11-Bis(trans-4-methoxycyclohexyl)-7-fluor-dispiro-[5.1.5.1]-tetradecan-14-on
3,11-Bis(trans-4-ethoxycyclohexyl)-7-fluor-dispiro-[5.1.5.1]-tetradecan-14-on
3,11-Bis(trans-4-propoxycyclohexyl)-7-fluor-dispiro-[5.1.5.1]-tetradecan-14-on
3,11-Bis(trans-4-butoxycyclohexyl)-7-fluor-dispiro-[5.1.5.1]-tetradecan-14-on
3,11-Bis(trans-4-pentoxycyclohexyl)-7-fluor-dispiro-[5.1.5.1]-tetradecan-14-on

3,11-Bis(trans-4-hexoxycyclohexyl)-7-fluor-dispiro-[5.1.5.1]-tetradecan-14-on
3,11-Bis(trans-4-heptoxycyclohexyl)-7-fluor-dispiro-[5.1.5.1]-tetradecan-14-on
3,11-Bis(trans-4-octoxycyclohexyl)-7-fluor-dispiro-[5.1.5.1]-tetradecan-14-on
3,11-Bis(trans-4-nonoxycyclohexyl)-7-fluor-dispiro-[5.1.5.1]-tetradecan-14-on
3,11-Bis(trans-4-decoxycyclohexyl)-7-fluor-dispiro-[5.1.5.1]-tetradecan-14-on
3,11-Bis(4-methylphenyl)-7-fluor-dispiro-[5.1.5.1]-tetradecan-14-on
3,11-Bis(4-ethylphenyl)-7-fluor-dispiro-[5.1.5.1]-tetradecan-14-on
3,11-Bis(4-propylphenyl)-7-fluor-dispiro-[5.1.5.1]-tetradecan-14-on
3,11-Bis(4-butylphenyl)-7-fluor-dispiro-[5.1.5.1]-tetradecan-14-on
3,11-Bis(4-pentylphenyl)-7-fluor-dispiro-[5.1.5.1]-tetradecan-14-on
3,11-Bis(4-hexylphenyl)-7-fluor-dispiro-[5.1.5.1]-tetradecan-14-on
3,11-Bis(4-heptylphenyl)-7-fluor-dispiro-[5.1.5.1]-tetradecan-14-on
3,11-Bis(4-octylphenyl)-7-fluor-dispiro-[5.1.5.1]-tetradecan-14-on
3,11-Bis(4-nonylphenyl)-7-fluor-dispiro-[5.1.5.1]-tetradecan-14-on
3,11-Bis(4-decylphenyl)-7-fluor-dispiro-[5.1.5.1]-tetradecan-14-on
3,11-Bis(4-methoxyphenyl)-7-fluor-dispiro-[5.1.5.1]-tetradecan-14-on
3,11-Bis(4-ethoxyphenyl)-7-fluor-dispiro-[5.1.5.1]-tetradecan-14-on
3,11-Bis(4-propoxyphenyl)-7-fluor-dispiro-[5.1.5.1]-tetradecan-14-on
3,11-Bis(4-butoxyphenyl)-7-fluor-dispiro-[5.1.5.1]-tetradecan-14-on
3,11-Bis(4-pentoxyphenyl)-7-fluor-dispiro-[5.1.5.1]-tetradecan-14-on
3,11-Bis(4-hexoxyphenyl)-7-fluor-dispiro-[5.1.5.1]-tetradecan-14-on
3,11-Bis(4-heptoxyphenyl)-7-fluor-dispiro-[5.1.5.1]-tetradecan-14-on
3,11-Bis(4-octoxyphenyl)-7-fluor-dispiro-[5.1.5.1]-tetradecan-14-on
3,11-Bis(4-nonoxyphenyl)-7-fluor-dispiro-[5.1.5.1]-tetradecan-14-on
3,11-Bis(4-decoxyphenyl)-7-fluor-dispiro-[5.1.5.1]-tetradecan-14-on
3-(trans-4-Methylcyclohexyl)-11-(trans-4-ethylcyclohexyl)-7-fluor-dispiro-[5.1.5.1]-tetradecan-14-on
3-(trans-4-Ethylcyclohexyl)-11-(trans-4-pentylcyclohexyl)-7-fluor-dispiro-[5.1.5.1]-tetradecan-14-on
3-(trans-4-Propylcyclohexyl)-11-(trans-4-ethylcyclohexyl)-7-fluor-dispiro-[5.1.5.1]-tetradecan-14-on
3-(trans-4-Butylcyclohexyl)-11-(trans-4-ethylcyclohexyl)-7-fluor-dispiro-[5.1.5.1]-tetradecan-14-on
3-(trans-4-Hexylcyclohexyl)-11-(trans-4-ethylcyclohexyl)-7-fluor-dispiro-[5.1.5.1]-tetradecan-14-on
3-(trans-4-Ethylcyclohexyl)-11-(trans-4-heptylcyclohexyl)-7-fluor-dispiro-[5.1.5.1]-tetradecan-14-on
3-(trans-4-Octylcyclohexyl)-11-(trans-4-propylcyclohexyl)-7-fluor-dispiro-[5.1.5.1]-tetradecan-14-on
3-(trans-4-Butylcyclohexyl)-11-(trans-4-nonylcyclohexyl)-7-fluor-dispiro-[5.1.5.1]-tetradecan-14-on
3-(trans-4-Decylcyclohexyl)-11-(trans-4-hexylcyclohexyl)-7-fluor-dispiro-[5.1.5.1]-tetradecan-14-on
3-(trans-4-Propylcyclohexyl)-11-trans-pentyl-7-fluor-dispiro-[5.1.5.1]-tetradecan-14-on
3-(trans-4-Propylcyclohexyl)-11-trans-ethyl-7-fluor-dispiro-[5.1.5.1]-tetradecan-14-on
3-(trans-4-Propylcyclohexyl)-11-trans-butyl-7-fluor-dispiro-[5.1.5.1]-tetradecan-14-on
3-(trans-4-Propylcyclohexyl)-11-trans-propyl-7-fluor-dispiro-[5.1.5.1]-tetradecan-14-on
3-(trans-4-Propylcyclohexyl)-11-trans-hexyl-7-fluor-dispiro-[5.1.5.1]-tetradecan-14-on
3-(trans-4-Butylcyclohexyl)-11-trans-butyl-7-fluor-dispiro-[5.1.5.1]-tetradecan-14-on
3-(trans-4-Butylcyclohexyl)-11-trans-ethyl-7-fluor-dispiro-[5.1.5.1]-tetradecan-14-on
3-(trans-4-Butylcyclohexyl)-11-trans-propyl-7-fluor-dispiro-[5.1.5.1]-tetradecan-14-on
3-(trans-4-Butylcyclohexyl)-11-trans-pentyl-7-fluor-dispiro-[5.1.5.1]-tetradecan-14-on
3-(trans-4-Butylcyclohexyl)-11-trans-hexyl-7-fluor-dispiro-[5.1.5.1]-tetradecan-14-on
3-(trans-4-Pentylcyclohexyl)-11-trans-ethyl-7-fluor-dispiro-[5.1.5.1]-tetradecan-14-on
3-(trans-4-Pentylcyclohexyl)-11-trans-propyl-7-fluor-dispiro-[5.1.5.1]-tetradecan-14-on
3-(trans-4-Pentylcyclohexyl)-11-trans-butyl-7-fluor-dispiro-[5.1.5.1]-tetradecan-14-on
3-(trans-4-Pentylcyclohexyl)-11-trans-pentyl-7-fluor-dispiro-[5.1.5.1]-tetradecan-14-on
3-(trans-4-Pentylcyclohexyl)-11-trans-hexyl-7-fluor-dispiro-[5.1.5.1]-tetradecan-14-on
3-(trans-4-Hexylcyclohexyl)-11-trans-ethyl-7-fluor-dispiro-[5.1.5.1]-tetradecan-14-on
3-(trans-4-Hexylcyclohexyl)-11-trans-propyl-7-fluor-dispiro-[5.1.5.1]-tetradecan-14-on
3-(trans-4-Hexylcyclohexyl)-11-trans-butyl-7-fluor-dispiro-[5.1.5.1]-tetradecan-14-on
3-(trans-4-Hexylcyclohexyl)-11-trans-pentyl-7-fluor-dispiro-[5.1.5.1]-tetradecan-14-on
3-(trans-4-Hexylcyclohexyl)-11-trans-hexyl-7-fluor-dispiro-[5.1.5.1]-tetradecan-14-on
3-(trans-4-Hexylcyclohexyl)-11-trans-heptyl-7-fluor-dispiro-[5.1.5.1]-tetradecan-14-on
3-(trans-4-Ethylcyclohexyl)-11-trans-pentyl-7-fluor-dispiro-[5.1.5.1]-tetradecan-14-on
3-(trans-4-Ethylcyclohexyl)-11-trans-hexyl-7-fluor-dispiro-[5.1.5.1]-tetradecan-14-on
3-(trans-4-Ethylcyclohexyl)-11-trans-heptyl-7-fluor-dispiro-[5.1.5.1]-tetradecan-14-on
3-(trans-4-Heptylcyclohexyl)-11-trans-propyl-7-fluor-dispiro-[5.1.5.1]-tetradecan-14-on
3-(trans-4-Heptylcyclohexyl)-11-trans-butyl-7-fluor-dispiro-[5.1.5.1]-tetradecan-14-on
3-(trans-4-Heptylcyclohexyl)-11-trans-pentyl-7-fluor-dispiro-[5.1.5.1]-tetradecan-14-on
3-(trans-4-Methylcyclohexyl)-11-trans-butyl-7-fluor-dispiro-[5.1.5.1]-tetradecan-14-on
3-(trans-4-Methylcyclohexyl)-11-trans-pentyl-7-fluor-dispiro-[5.1.5.1]-tetradecan-14-on
3-(trans-4-Methylcyclohexyl)-11-trans-hexyl-7-fluor-dispiro-[5.1.5.1]-tetradecan-14-on
3-[trans-4-(p-Propylcyclohexyl]-11-trans-pentyl-7-fluor-dispiro-[5.1.5.1]-tetradecan-14-on
3-[trans-4-(p-Propylcyclohexyl]-11-trans-propyl-7-fluor-dispiro-[5.1.5.1]-tetradecan-14-on
3-[trans-4-(p-Propylcyclohexyl]-11-trans-ethyl-7-fluor-dispiro-[5.1.5.1]-tetradecan-14-on
3-[trans-4-(p-Propylcyclohexyl]-11-trans-butyl-7-fluor-dispiro-[5.1.5.1]-tetradecan-14-on
3-[trans-4-(p-Pentylcyclohexyl]-11-trans-pentyl-7-fluor-dispiro-[5.1.5.1]-tetradecan-14-on
3-[trans-4-(p-Pentylcyclohexyl]-11-trans-propyl-7-fluor-dispiro-[5.1.5.1]-tetradecan-14-on
3-[trans-4-(p-Ethylcyclohexyl]-11-trans-butyl-7-fluor-dispiro-[5.1.5.1]-tetradecan-14-on
3-[trans-4-(p-Methylcyclohexyl]-11-trans-propyl-7-fluor-dispiro-[5.1.5.1]-tetradecan-14-on
3-[trans-4-(p-Pentylcyclohexyl]-11-trans-methyl-7-fluor-dispiro-[5.1.5.1]-tetradecan-14-on
3-[trans-4-(p-Heptylcyclohexyl]-11-trans-heptyl-7-fluor-dispiro-[5.1.5.1]-tetradecan-14-on

*Beispiel 11:*

Ein Gemisch aus 1,8 g 3,11-Dipentyl-7-oxo-dispiro-[5.1.5.1]-tetradecan-14-dithion (erhältlich aus dem entsprechenden Diketon durch Umsetzung mit $P_4S_{10}$ in Pyridin) und 25 ml THF werden bei Raumtemperatur zu einer gut gerührten Aufschlämmung von frischem Raney-Nickel in THF gegeben. Man rührt 5 Stunden nach und arbeitet wie üblich auf. Nach Umkristallisation aus Ethanol erhält man 3,11-trans-Dipentyl-dispiro-[5.1.5.1]-tetradecan-7-on mit Fp. 66° und Kp. 111°.

Analog werden hergestellt:

3,11-trans-Dimethyl-dispiro-[5.1.5.1]-tetradecan-7-on
3,11-trans-Diethyl-dispiro-[5.1.5.1]-tetradecan-7-on
3,11-trans-Dipropyl-dispiro-[5.1.5.1]-tetradecan-7-on
3,11-trans-Dibutyl-dispiro-[5.1.5.1]-tetradecan-7-on
3,11-trans-Dihexyl-dispiro-[5.1.5.1]-tetradecan-7-on
3,11-trans-Diheptyl-dispiro-[5.1.5.1]-tetradecan-7-on
3,11-trans-Dioctyl-dispiro-[5.1.5.1]-tetradecan-7-on
3,11-trans-Dinonyl-dispiro-[5.1.5 1]-tetradecan-7-on
3,11-trans-Didecyl-dispiro-[5.1.5.1]-tetradecan-7-on
3,11-trans-Dimethoxy-dispiro-[5.1.5.1]-tetradecan-7-on
3,11-trans-Diethoxy-dispiro-[5.1.5.1]-tetradecan-7-on
3,11-trans-Dipropoxy-dispiro-[5.1.5.1]-tetradecan-7-on
3,11-trans-Dibutoxy-dispiro-[5.1.5.1]-tetradecan-7-on
3,11-trans-Dipentoxy-dispiro-[5.1.5.1]-tetradecan-7-on
3,11-trans-Dihexoxy-dispiro-[5.1.5.1]-tetradecan-7-on
3,11-trans-Diheptoxy-dispiro-[5.1.5.1]-tetradecan-7-on
3,11-trans-Dioctoxy-dispiro-[5.1.5.1]-tetradecan-7-on
3,11-trans-Dinonoxy-dispiro-[5.1.5.1]-tetradecan-7-on
3,11-trans-Didecoxy-dispiro-[5.1.5.1]-tetradecan-7-on
3-trans-Methyl-11-trans-propyl-dispiro-[5.1.5.1]-tetradecan-7-on
3-trans-Propyl-11-trans-pentyl-dispiro-[5.1.5.1]-tetradecan-7-on
3-trans-Butyl-11-trans-heptyl-dispiro-[5.1.5.1]-tetradecan-7-on
3-trans-Pentyl-11-trans-heptyl-dispiro-[5.1.5.1]-tetradecan-7-on
3-trans-Octyl-11-trans-propyl-dispiro-[5.1.5.1]-tetradecan-7-on
3-trans-Propyl-11-trans-ethyl-dispiro-[5.1.5.1]-tetradecan-7-on
3,11-Bis-(trans-4-methylcyclohexyl)-dispiro-[5.1.5.1]-tetradecan-7-on
3,11-Bis-(trans-4-ethylcyclohexyl)-dispiro-[5.1.5.1]-tetradecan-7-on
3,11-Bis-(trans-4-propylcyclohexyl)-dispiro-[5.1.5.1]-tetradecan-7-on, K 338° I
3,11-Bis-(trans-4-butylcyclohexyl)-dispiro-[5.1.5.1]-tetradecan-7-on
3,11-Bis-(trans-4-pentylcyclohexyl)-dispiro-[5.1.5.1]-tetradecan-7-on
3,11-Bis-(trans-4-hexylcyclohexyl)-dispiro-[5.1.5.1]-tetradecan-7-on
3,11-Bis-(trans-4-heptylcyclohexyl)-dispiro-[5.1.5.1]-tetradecan-7-on
3,11-Bis-(trans-4-octylcyclohexyl)-dispiro-[5.1.5.1]-tetradecan-7-on
3,11-Bis-(trans-4-nonylcyclohexyl)-dispiro-[5.1.5.1]-tetradecan-7-on
3,11-Bis-(trans-4-decylcyclohexyl)-dispiro-[5.1.5.1]-tetradecan-7-on
3,11-Bis-(trans-4-methoxycyclohexyl)-dispiro-[5.1.5.1]-tetradecan-7-on
3,11-Bis-(trans-4-ethoxycyclohexyl)-dispiro-[5.1.5.1]-tetradecan-7-on
3,11-Bis-(trans-4-propoxycyclohexyl)-dispiro-[5.1.5.1]-tetradecan-7-on
3,11-Bis-(trans-4-butoxycyclohexyl)-dispiro-[5.1.5.1]-tetradecan-7-on

3,11-Bis-(trans-4-pentoxycyclohexyl)-dispiro-[5.1.5.1]-tetradecan-7-on
3,11-Bis-(trans-4-hexoxycyclohexyl)-dispiro-[5.1.5.1]-tetradecan-7-on
3,11-Bis-(trans-4-heptoxycyclohexyl)-dispiro-[5.1.5.1]-tetradecan-7-on
3,11-Bis-(trans-4-octoxycyclohexyl)-dispiro-[5.1.5.1]-tetradecan-7-on
3,11-Bis-(trans-4-nonoxycyclohexyl)-dispiro-[5.1.5.1]-tetradecan-7-on
3,11-Bis-(trans-4-decoxycyclohexyl)-dispiro-[5.1.5.1]-tetradecan-7-on
3-(trans-4-Methylcyclohexyl)-11-(trans-4-ethylcyclohexyl)-dispiro-[5.1.5.1]-tetradecan-7-on
3-(trans-4-Ethylcyclohexyl)-11-(trans-4-pentylcyclohexyl)-dispiro-[5.1.5.1]-tetradecan-7-on
3-(trans-4-Propylcyclohexyl)-11-(trans-4-pentylcyclohexyl)-dispiro-[5.1.5.1]-tetradecan-7-on
3-(trans-4-Butylcyclohexyl)-11-(trans-4-ethylcyclohexyl)-dispiro-[5.1.5.1]-tetradecan-7-on
3-(trans-4-Pentylcyclohexyl)-11-(trans-4-heptylcyclohexyl)-dispiro-[5.1.5.1]-tetradecan-7-on
3-(trans-4-Octylcyclohexyl)-11-(trans-4-propylcyclohexyl)-dispiro-[5.1.5.1]-tetradecan-7-on
3-(trans-4-Propylcyclohexyl)-11-(trans-4-ethylcyclohexyl)-dispiro-[5.1.5.1]-tetradecan-7-on
3-(trans-4-Propylcyclohexyl)-11-trans-propyl-dispiro-[5.1.5.1]-tetradecan-7-on
3-(trans-4-Propylcyclohexyl)-11-trans-ethyl-dispiro-[5.1.5.1]-tetradecan-7-on
3-(trans-4-Propylcyclohexyl)-11-trans-butyl-dispiro-[5.1.5.1]-tetradecan-7-on
3-(trans-4-Propylcyclohexyl)-11-trans-pentyl-dispiro-[5.1.5.1]-tetradecan-7-on
3-(trans-4-Propylcyclohexyl)-11-trans-hexyl-dispiro-[5.1.5.1]-tetradecan-7-on
3-(trans-4-Propylcyclohexyl)-11-trans-heptyl-dispiro-[5.1.5.1]-tetradecan-7-on
3-(trans-4-Butylcyclohexyl)-11-trans-propyl-dispiro-[5.1.5.1]-tetradecan-7-on
3-(trans-4-Butylcyclohexyl)-11-trans-ethyl-dispiro-[5.1.5.1]-tetradecan-7-on
3-(trans-4-Butylcyclohexyl)-11-trans-butyl-dispiro-[5.1.5.1]-tetradecan-7-on
3-(trans-4-Butylcyclohexyl)-11-trans-pentyl-dispiro-[5.1.5.1]-tetradecan-7-on
3-(trans-4-Butylcyclohexyl)-11-trans-hexyl-dispiro-[5.1.5.1]-tetradecan-7-on
3-(trans-4-Butylcyclohexyl)-11-trans-heptyl-dispiro-[5.1.5.1]-tetradecan-7-on
3-(trans-4-Pentylcyclohexyl)-11-trans-propyl-dispiro-[5.1.5.1]-tetradecan-7-on
3-(trans-4-Pentylcyclohexyl)-11-trans-ethyl-dispiro-[5.1.5.1]-tetradecan-7-on
3-(trans-4-Pentylcyclohexyl)-11-trans-butyl-dispiro-[5.1.5.1]-tetradecan-7-on
3-(trans-4-Pentylcyclohexyl)-11-trans-pentyl-dispiro-[5.1.5.1]-tetradecan-7-on
3-(trans-4-Pentylcyclohexyl)-11-trans-hexyl-dispiro-[5.1.5.1]-tetradecan-7-on
3-(trans-4-Pentylcyclohexyl)-11-trans-heptyl-dispiro-[5.1.5.1]-tetradecan-7-on
3-(trans-4-Ethylcyclohexyl)-11-trans-propyl-dispiro-[5.1.5.1]-tetradecan-7-on
3-(trans-4-Ethylcyclohexyl)-11-trans-ethyl-dispiro-[5.1.5.1]-tetradecan-7-on
3-(trans-4-Ethylcyclohexyl)-11-trans-butyl-dispiro-[5.1.5.1]-tetradecan-7-on
3-(trans-4-Ethylcyclohexyl)-11-trans-pentyl-dispiro-[5.1.5.1]-tetradecan-7-on
3-(trans-4-Ethylcyclohexyl)-11-trans-hexyl-dispiro-[5.1.5.1]-tetradecan-7-on
3-(trans-4-Ethylcyclohexyl)-11-trans-heptyl-dispiro-[5.1.5.1]-tetradecan-7-on
3-(trans-4-Methylcyclohexyl)-11-trans-propyl-dispiro-[5.1.5.1]-tetradecan-7-on
3-(trans-4-Methylcyclohexyl)-11-trans-ethyl-dispiro-[5.1.5.1]-tetradecan-7-on
3-(trans-4-Methylcyclohexyl)-11-trans-butyl-dispiro-[5.1.5.1]-tetradecan-7-on
3-(trans-4-Methylcyclohexyl)-11-trans-pentyl-dispiro-[5.1.5.1]-tetradecan-7-on
3-(trans-4-Methylcyclohexyl)-11-trans-hexyl-dispiro-[5.1.5.1]-tetradecan-7-on
3-(trans-4-Methylcyclohexyl)-11-trans-heptyl-dispiro-[5.1.5.1]-tetradecan-7-on
3-(trans-4-Hexylcyclohexyl)-11-trans-propyl-dispiro-[5.1.5.1]-tetradecan-7-on
3-(trans-4-Hexylcyclohexyl)-11-trans-ethyl-dispiro-[5.1.5.1]-tetradecan-7-on
3-(trans-4-Hexylcyclohexyl)-11-trans-butyl-dispiro-[5.1.5.1]-tetradecan-7-on
3-(trans-4-Hexylcyclohexyl)-11-trans-pentyl-dispiro-[5.1.5.1]-tetradecan-7-on
3-(trans-4-Hexylcyclohexyl)-11-trans-hexyl-dispiro-[5.1.5.1]-tetradecan-7-on
3-(trans-4-Hexylcyclohexyl)-11-trans-heptyl-dispiro-[5.1.5.1]-tetradecan-7-on
3-(trans-4-Heptylcyclohexyl)-11-trans-propyl-dispiro-[5.1.5.1]-tetradecan-7-on
3-(trans-4-Heptylcyclohexyl)-11-trans-ethyl-dispiro-[5.1.5.1]-tetradecan-7-on
3-(trans-4-Heptylcyclohexyl)-11-trans-butyl-dispiro-[5.1.5.1]-tetradecan-7-on
3-(trans-4-Heptylcyclohexyl)-11-trans-pentyl-dispiro-[5.1.5.1]-tetradecan-7-on
3-(trans-4-Heptylcyclohexyl)-11-trans-hexyl-dispiro-[5.1.5.1]-tetradecan-7-on
3-(trans-4-Heptylcyclohexyl)-11-trans-heptyl-dispiro-[5.1.5.1]-tetradecan-7-on
3-[trans-4-(p-Propylcyclohexyl)cyclohexyl]-11-trans-pentyldispiro-[5.1.5.1]-tetradecan-7-on
3-[trans-4-(p-Propylcyclohexyl)cyclohexyl]-11-trans-ethyldispiro-[5.1.5.1]-tetradecan-7-on
3-[trans-4-(p-Propylcyclohexyl)cyclohexyl]-11-trans-propyldispiro-[5.1.5.1]-tetradecan-7-on
3-[trans-4-(p-Propylcyclohexyl)cyclohexyl]-11-trans-butyldispiro-[5.1.5.1]-tetradecan-7-on
3-[trans-4-(p-Propylcyclohexyl)cyclohexyl]-11-trans-hexyldispiro-[5.1.5.1]-tetradecan-7-on
3-[trans-4-(p-Propylcyclohexyl)cyclohexyl]-11-trans-heptyldispiro-[5.1.5.1]-tetradecan-7-on
3-[trans-4-(p-Ethylcyclohexyl)cyclohexyl]-11-trans-pentyldispiro-[5.1.5.1]-tetradecan-7-on
3-[trans-4-(p-Ethylcyclohexyl)cyclohexyl]-11-trans-ethyldispiro-[5.1.5.1]-tetradecan-7-on
3-[trans-4-(p-Ethylcyclohexyl)cyclohexyl]-11-trans-propyldispiro-[5.1.5.1]-tetradecan-7-on
3-[trans-4-(p-Ethylcyclohexyl)cyclohexyl]-11-trans-butyldispiro-[5.1.5.1]-tetradecan-7-on
3-[trans-4-(p-Ethylcyclohexyl)cyclohexyl]-11-trans-hexyldispiro-[5.1.5.1]-tetradecan-7-on
3-[trans-4-(p-Ethylcyclohexyl)cyclohexyl]-11-trans-heptyldispiro-[5.1.5.1]-tetradecan-7-on
3-[trans-4-(p-Butylcyclohexyl)cyclohexyl]-11-trans-pentyldispiro-[5.1.5.1]-tetradecan-7-on
3-[trans-4-(p-Butylcyclohexyl)cyclohexyl]-11-trans-ethyldispiro-[5.1.5.1]-tetradecan-7-on
3-[trans-4-(p-Butylcyclohexyl)cyclohexyl]-11-trans-propyldispiro-[5.1.5.1]-tetradecan-7-on
3-[trans-4-(p-Butylcyclohexyl)cyclohexyl]-11-trans-butyldispiro-[5.1.5.1]-tetradecan-7-on
3-[trans-4-(p-Butylcyclohexyl)cyclohexyl]-11-trans-hexyldispiro-[5.1.5.1]-tetradecan-7-on
3-[trans-4-(p-Butylcyclohexyl)cyclohexyl]-11-trans-heptydispiro-[5.1.5.1]-tetradecan-7-on
3-[trans-4-(p-Pentylcyclohexyl)cyclohexyl]-11-trans-pentyldispiro-[5.1.5.1]-tetradecan-7-on
3-[trans-4-(p-Pentylcyclohexyl)cyclohexyl]-11-trans-ethyldispiro-[5.1.5.1]-tetradecan-7-on
3-[trans-4-(p-Pentylcyclohexyl)cyclohexyl]-11-trans-propyldispiro-[5.1.5.1]-tetradecan-7-on
3-[trans-4-(p-Pentylcyclohexyl)cyclohexyl]-11-trans-butyldispiro-[5.1.5.1]-tetradecan-7-on
3-[trans-4-(p-Pentylcyclohexyl)cyclohexyl]-11-trans-hexyldispiro-[5.1.5.1]-tetradecan-7-on
3-[trans-4-(p-Pentylcyclohexyl)cyclohexyl]-11-trans-heptyldispiro-[5.1.5.1]-tetradecan-7-on
3-[trans-4-(p-Hexylcyclohexyl)cyclohexyl]-11-trans-pentyldispiro-[5.1.5.1]-tetradecan-7-on
3-[trans-4-(p-Hexylcyclohexyl)cyclohexyl]-11-trans-ethyldispiro-[5.1.5.1]-tetradecan-7-on
3-[trans-4-(p-Hexylcyclohexyl)cyclohexyl]-11-trans-propyldispiro-[5.1.5.1]-tetradecan-7-on
3-[trans-4-(p-Hexylcyclohexyl)cyclohexyl]-11-trans-butyldispiro-[5.1.5.1]-tetradecan-7-on
3-[trans-4-(p-Hexylcyclohexyl)cyclohexyl]-11-trans-hexyldispiro-[5.1.5.1]-tetradecan-7-on
3-[trans-4-(p-Hexylcyclohexyl)cyclohexyl]-11-trans-heptyldispiro-[5.1.5.1]-tetradecan-7-on
3-[trans-4-(p-Heptylcyclohexyl)cyclohexyl]-11-trans-pentyldispiro-[5.1.5.1]-tetradecan-7-on
3-[trans-4-(p-Heptylcyclohexyl)cyclohexyl]-11-trans-ethyldispiro-[5.1.5.1]-tetradecan-7-on
3-[trans-4-(p-Heptylcyclohexyl)cyclohexyl]-11-trans-propyldispiro-[5.1.5.1]-tetradecan-7-on
3-[trans-4-(p-Heptylcyclohexyl)cyclohexyl]-11-trans-butyldispiro-[5.1.5.1]-tetradecan-7-on
3-[trans-4-(p-Heptylcyclohexyl)cyclohexyl]-11-trans-hexyldispiro-[5.1.5.1]-tetradecan-7-on
3-[trans-4-(p-Heptylcyclohexyl)cyclohexyl]-11-trans-heptyldispiro-[5.1.5.1]-tetradecan-7-on

*Beispiel 12:*

Analog Beispiel 2 erhält man ausgehend von 3,11-Bis-(trans-4-propylcyclohexyl)-dispiro-[5.1.5.1]-tetradecan-7-on das entsprechende 3,11-Bis-(trans-4-propylcyclohexyl)-7,7-dichlor-dispiro-[5.1.5.1]-tetradecan mit K 172° N 266° I.

Analog werden hergestellt:

3,11-Bis-(trans-4-methylcyclohexyl)-7,7-dichlor-dispiro-[5.1.5.1]-tetradecan
3,11-Bis-(trans-4-ethylcyclohexyl)-7,7-dichlor-dispiro-[5.1.5.1]-tetradecan
3,11-Bis-(trans-4-butylcyclohexyl)-7,7-dichlor-dispiro-[5.1.5.1]-tetradecan
3,11-Bis-(trans-4-pentylcyclohexyl)-7,7-dichlor-dispiro-[5.1.5.1]-tetradecan
3,11-Bis-(trans-4-hexylcyclohexyl)-7,7-dichlor-dispiro-[5.1.5.1]-tetradecan
3,11-Bis-(trans-4-heptylcyclohexyl)-7,7-dichlor-dispiro-[5.1.5.1]-tetradecan
3,11-Bis-(trans-4-octylcyclohexyl)-7,7-dichlor-dispiro-[5.1.5.1]-tetradecan
3,11-Bis-(trans-4-nonylcyclohexyl)-7,7-dichlor-dispiro-[5.1.5.1]-tetradecan
3,11-Bis-(trans-4-decylcyclohexyl)-7,7-dichlor-dispiro-[5.1.5.1]-tetradecan
3,11-Bis-(trans-4-methoxycyclohexyl)-7,7-dichlor-dispiro-[5.1.5.1]-tetradecan
3,11-Bis-(trans-4-ethoxycyclohexyl)-7,7-dichlor-dispiro-[5.1.5.1]-tetradecan
3,11-Bis-(trans-4-propoxycyclohexyl)-7,7-dichlor-dispiro-[5.1.5.1]-tetradecan
3,11-Bis-(trans-4-butoxycyclohexyl)-7,7-dichlor-dispiro-[5.1.5.1]-tetradecan
3,11-Bis-(trans-4-pentoxycyclohexyl)-7,7-dichlor-dispiro-[5.1.5.1]-tetradecan
3,11-Bis-(trans-4-hexoxycyclohexyl)-7,7-dichlor-dispiro-[5.1.5.1]-tetradecan
3,11-Bis-(trans-4-heptoxycyclohexyl)-7,7-dichlor-dispiro-[5.1.5.1]-tetradecan
3,11-Bis-(trans-4-octoxycyclohexyl)-7,7-dichlor-dispiro-[5.1.5.1]-tetradecan
3,11-Bis-(trans-4-nonoxycyclohexyl)-7,7-dichlor-dispiro-[5.1.5.1]-tetradecan
3,11-Bis-(trans-4-decoxycyclohexyl)-7,7-dichlor-dispiro-[5.1.5.1]-tetradecan
3,11-Bis-(4-propylphenyl)-7,7-dichlor-dispiro-[5.1.5.1]-tetradecan
3,11-Bis-(4-methylphenyl)-7,7-dichlor-dispiro-[5.1.5.1]-tetradecan
3,11-Bis-(4-ethylphenyl)-7,7-dichlor-dispiro-[5.1.5.1]-tetradecan
3,11-Bis-(4-butylphenyl)-7,7-dichlor-dispiro-[5.1.5.1]-tetradecan
3,11-Bis-(4-pentylphenyl)-7,7-dichlor-dispiro-[5.1.5.1]-tetradecan
3,11-Bis-(4-hexylphenyl)-7,7-dichlor-dispiro-[5.1.5.1]-tetradecan
3,11-Bis-(4-heptylphenyl)-7,7-dichlor-dispiro-[5.1.5.1]-tetradecan
3,11-Bis-(4-octylphenyl)-7,7-dichlor-dispiro-[5.1.5.1]-tetradecan
3,11-Bis-(4-nonylphenyl)-7,7-dichlor-dispiro-[5.1.5.1]-tetradecan
3,11-Bis-(4-decylphenyl)-7,7-dichlor-dispiro-[5.1.5.1]-tetradecan
3,11-Bis-(4-methoxyphenyl)-7,7-dichlor-dispiro-[5.1.5.1]-tetradecan
3,11-Bis-(4-ethoxyphenyl)-7,7-dichlor-dispiro-[5.1.5.1]-tetradecan
3,11-Bis-(4-propoxyphenyl)-7,7-dichlor-dispiro-[5.1.5.1]-tetradecan
3,11-Bis-(4-butoxyphenyl)-7,7-dichlor-dispiro-[5.1.5.1]-tetradecan
3,11-Bis-(4-pentoxyphenyl)-7,7-dichlor-dispiro-[5.1.5.1]-tetradecan
3,11-Bis-(4-hexoxylphenyl)-7,7-dichlor-dispiro-[5.1.5.1]-tetradecan
3,11-Bis-(4-heptoxyphenyl)-7,7-dichlor-dispiro-[5.1.5.1]-tetradecan
3,11-Bis-(4-octoxyphenyl)-7,7-dichlor-dispiro-[5.1.5.1]-tetradecan
3,11-Bis-(4-nonoxyphenyl)-7,7-dichlor-dispiro-[5.1.5.1]-tetradecan
3,11-Bis-(4-decoxyphenyl)-7,7-dichlor-dispiro-[5.1.5.1]-tetradecan
3,11-trans-Dipropyl-7,7-dichlor-dispiro-[5.1.5.1]-tetradecan
3,11-trans-Dimethyl-7,7-dichlor-dispiro-[5.1.5.1]-tetradecan
3,11-trans-Diethyl-7,7-dichlor-dispiro-[5.1.5.1]-tetradecan
3,11-trans-Dibutyl-7,7-dichlor-dispiro-[5.1.5.1]-tetradecan
3,11-trans-Dipentyl-7,7-dichlor-dispiro-[5.1.5.1]-tetradecan
3,11-trans-Dihexyl-7,7-dichlor-dispiro-[5.1.5.1]-tetradecan
3,11-trans-Diheptyl-7,7-dichlor-dispiro-[5.1.5.1]-tetradecan
3,11-trans-Dioctyl-7,7-dichlor-dispiro-[5.1.5.1]-tetradecan
3,11-trans-Dinonyl-7,7-dichlor-dispiro-[5.1.5.1]-tetradecan
3,11-trans-Didecyl-7,7-dichlor-dispiro-[5.1.5.1]-tetradecan
3,11-trans-Dimethoxy-7,7-dichlor-dispiro-[5.1.5.1]-tetradecan
3,11-trans-Diethoxy-7,7-dichlor-dispiro-[5.1.5.1]-tetradecan
3,11-trans-Dipropoxy-7,7-dichlor-dispiro-[5.1.5.1]-tetradecan
3,11-trans-Dibutoxy-7,7-dichlor-dispiro-[5.1.5.1]-tetradecan
3,11-trans-Dipentoxy-7,7-dichlor-dispiro-[5.1.5.1]-tetradecan
3,11-trans-Dihexoxy-7,7-dichlor-dispiro-[5.1.5.1]-tetradecan
3,11-trans-Diheptoxy-7,7-dichlor-dispiro-[5.1.5.1]-tetradecan
3,11-trans-Dioctoxy-7,7-dichlor-dispiro-[5.1.5.1]-tetradecan
3,11-trans-Dinonoxy-7,7-dichlor-dispiro-[5.1.5.1]-tetradecan
3,11-trans-Didecoxy-7,7-dichlor-dispiro-[5.1.5.1]-tetradecan
3-(trans-4-Methylcyclohexyl)-11-(trans-4-ethylcyclohexyl)-7,7-dichlor-dispiro-[5.1.5.1]-tetradecan
3-(trans-4-Ethylcyclohexyl)-11-(trans-4-pentylcyclohexyl)-7,7-dichlor-dispiro-[5.1.5.1]-tetradecan
3-(trans-4-Propylcyclohexyl)-11-(trans-4-ethylcyclohexyl)-7,7-dichlor-dispiro-[5.1.5.1]-tetradecan
3-(trans-4-Butylcyclohexyl)-11-(trans-4-ethylcyclohexyl)-7,7-dichlor-dispiro-[5.1.5.1]-tetradecan
3-(trans-4-Hexylcyclohexyl)-11-(trans-4-ethylcyclohexyl)-7,7-dichlor-dispiro-[5.1.5.1]-tetradecan
3-(trans-4-Ethylcyclohexyl)-11-(trans-4-heptylcyclohexyl)-7,7-dichlor-dispiro-[5.1.5.1]-tetradecan
3-(trans-4-Octylcyclohexyl)-11-(trans-4-propylethylcyclohexyl)-7,7-dichlor-dispiro-[5.1.5.1]-tetradecan
3-(trans-4-Butylcyclohexyl)-11-(trans-4-nonylcyclohexyl)-7,7-dichlor-dispiro-[5.1.5.1]-tetradecan
3-(trans-4-Decylcyclohexyl)-11-(trans-4-hexylcyclohexyl)-7,7-dichlor-dispiro-[5.1.5.1]-tetradecan
3-trans-Methyl-11-trans-propyl-7,7-dichlor-dispiro-[5.1.5.1]-tetradecan
3-trans-Propyl-11-trans-pentyl-7,7-dichlor-dispiro-[5.1.5.1]-tetradecan
3-trans-Butyl-11-trans-ethyl-7,7-dichlor-dispiro-[5.1.5.1]-tetradecan
3-trans-Pentyl-11-trans-hexyl-7,7-dichlor-dispiro-[5.1.5.1]-tetradecan
3-trans-Octyl-11-trans-pentyl-7,7-dichlor-dispiro-[5.1.5.1]-tetradecan

3-trans-Propyl-11-trans-ethyl-7,7-dichlor-dispiro-[5.1.5.1]-tetradecan

Folgende Beispiele betreffen flüssigkristalline Phase.

*Beispiel A:*

Eine flüssigkristalline Phase, bestehend aus
17% p-trans-4-Propylcyclohexyl-benzonitril,
16% trans-1-p-Ethoxyphenyl-4-propyl-cyclohexan,
23% p-trans-4-Pentylcyclohexyl-benzonitril,
12% trans-1-p-Butoxyphenyl-4-propyl-cyclohexan,
17% 4-Ethyl-4'-(trans-4-pentylcyclohexyl)-biphenyl, und
15% 3,11-Bis(trans-4-propylcyclohexyl)-14,14-dichlor-dispiro-[5.1.5.1]-tetradecan-7-on
hat Kp. 85°.

*Beispiel B:*

Eine flüssigkristalline Phase, bestehend aus
15% p-trans-4-Propylcyclohexyl-benzonitril,
11% p-trans-4-Butylcyclohexyl-benzonitril,
21% p-trans-4-Pentylcyclohexyl-benzonitril,
15% 3,11-trans-Dipentyl-14,14-dichlordispiro-[5.1.5.1]-tetradecan-7-on,
21% 4-Ethyl-4'-(trans-4-pentylcyclohexyl)-biphenyl,
12% 4-(trans-4-Pentylcyclohexyl-4'-(trans-4-propylcyclohexyl)-biphenyl, und
5% 4-Cyan-4'-(trans-4-pentylcyclohexyl)-biphenyl
hat Kp. 98°.

**Patentansprüche**

1. Dispirotetradecane der Formel I

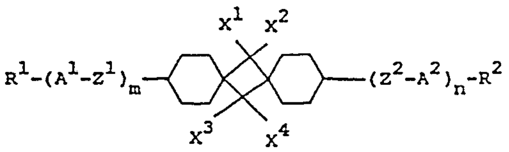 (I)

worin

$R^1$ und $R^2$ jeweils unabhängig voneinander Alkyl mit 1 bis 12 C-Atomen, worin auch eine oder mehrere nicht benachbarte $CH_2$-Gruppen durch $-O-$, $-CO-$, $-CO-O-$, $-O-CO$, $-O-COO-$ und/oder $-CH=CH-$ (trans) ersetzt sein können, einer der Reste $R^1$ und $R^2$ auch H, F, Cl, Br, J, CN, $NO_2$, NCS,

$A^1$ und $A^2$ jeweils unabhängig voneinander trans-1,4-Cyclohexylen, worin auch eine oder zwei nicht benachbarte $CH_2$-Gruppen durch $-O-$ und/oder $-S-$ ersetzt sein können oder 1,4-Phenylen, worin auch eine oder mehrere CH-Gruppen durch N ersetzt sein können, $A^1$ und $A^2$ gegebenenfalls auch lateral bzw. axial substituiert sein können durch F, Cl, CN, $CH_3$,

$Z^1$ und $Z^2$ jeweils unabhängig voneinander $-CO-O-$, $-O-CO$, $-CH_2CH_2-$, $-CH_2O-$, $-OCH_2-$ oder eine Einfachbindung,

m und n jeweils 0, 1 oder 2,

(m + n) 0, 1 oder 2,

$X^1$, $X^2$, $X^3$ und $X^4$ jeweils unbhängig voneinander H, F, Cl oder CN,

bedeuten, und eine oder beide der Gruppen $CX^1X^2$ und $CX^3X^4$ auch C=O bedeuten können, mit der Massgabe, dass (m + n) 1 oder 2 ist, wenn beide Gruppen $CX^1X^2$ und $CX^3X^4$ C=O bedeuten.

2. Verbindungen der Formel I nach Anspruch 1, dadurch gekennzeichnet, dass sie den Teilformeln Ia bis Id

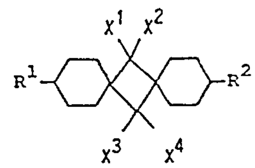 (Ia)

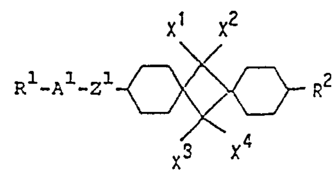 (Ib)

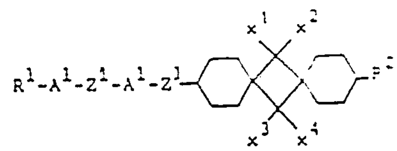 (Ic)

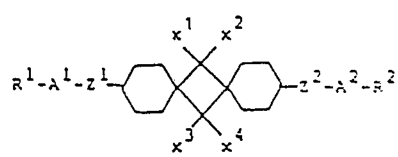 (Id)

entsprechen,

worin $R^1$, $A^1$, $Z^1$, $X^1$, $X^2$, $X^3$, $X^4$, $Z^2$, $A^2$ und $R^2$ die in Anspruch 1 angegebene Bedeutung haben.

3. Verbindungen nach einem der Ansprüche 1-2, dadurch gekennzeichnet, dass das Strukturelement

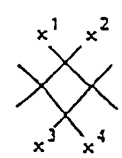

einer der Formeln 1-15

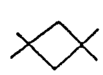 1 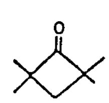 2 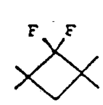 3 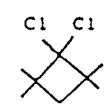 4

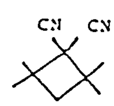 5 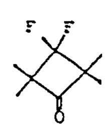 6 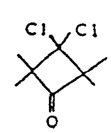 7 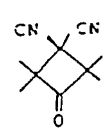 8

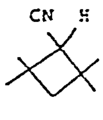
9

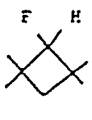
10

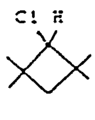
11

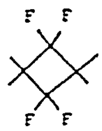
12

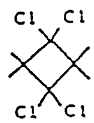
13

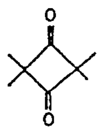
14

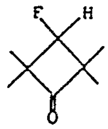
15

entspricht.

4. Verbindungen nach mindestens einem der Ansprüche 1 bis 3, worin $A^1$ und $A^2$ 1,4-Cyclohexylen oder 1,4-Phenylen ist.

5. Verbindungen nach mindestens einem der Ansprüche 1 bis 4, worin $Z^1$ und $Z^2$ jeweils unabhängig voneinander Einfachbindungen, $-CO-O-$ oder $-O-CO$ bedeuten.

6. Verbindungen nach mindestens einem der Ansprüche 1 bis 5, worin $X^1 = X^2 = X^3 = X^4 = H$ ist.

7. Verbindungen nach mindestens einem der Ansprüche 1 bis 5, worin eine der Gruppen $CX^1X^2$ und $CX^3X^4$ eine C=O-Gruppe ist.

8. Verbindungen nach mindestens einem der Ansprüche 1 bis 7, worin $R^1$ und $R^2$ Alkyl oder Alkoxy bedeuten.

9. Verfahren zur Herstellung von Dispirotetradecanen der Formel I nach Anspruch 1, dadurch gekennzeichnet, dass man entsprechende Ketene dimerisiert,

oder dass man in einer Verbindung, die sonst der Formel I entspricht, aber in 7,14-Position zwei C=O-Gruppen enthält, eine oder beide C=O-Gruppen mit einem reduzierenden Mittel behandelt,

oder dass man eine oder beide C=O-Gruppen durch Umsetzung mit einem organischen Säurehalogenid in die entsprechenden Halogenverbindungen überführt,

oder dass man eine Verbindung, die sonst der Formel I entspricht, aber an Stelle von H-Atomen eine oder mehrere reduzierbare Gruppen und/oder C-C-Bindungen enthält, mit einem reduzierenden Mittel behandelt,

oder dass man zur Herstellung von Estern der Formel I (worin $R^1$ und/oder $R^2$ eine Alkylgruppe bedeuten, worin eine oder mehrere $CH_2$-Gruppen durch $-O-CO$-Gruppen und/oder $-CO-O$-Gruppen ersetzt sind und/oder worin $Z^1$ und/oder $Z^2$ $-CO-O-$ oder $-O-CO-$ bedeuten) eine entsprechende Carbonsäure oder eines ihrer reaktionsfähigen Derivate mit einem entsprechenden Alkohol oder einem seiner reaktionsfähigen Derivate umsetzt,

oder dass man zur Herstellung von 1,3-Dioxanderivaten bzw. 1,3-Dithianderivaten der Formel I (worin $A^1$ und/oder $A^2$, 1,3-Dioxan-2,5-diyl bzw. 1,3-Dithian-2,5-diyl bedeutet) einen entsprechenden Aldehyd mit einem entsprechenden Diol bzw. Dithiol umsetzt,

oder dass man zur Herstellung von Ethern der Formel I (worin $R^1$ und/oder $R^2$ eine Alkylgruppe bedeutet, worin eine oder mehrere $CH_2$-Gruppen durch O-Atome ersetzt sind und/oder $Z^1$ und/oder $Z^2$ eine $-OCH_2-$ oder $-CH_2O$-Gruppe ist) eine entsprechende Hydroxyverbindung verethert,

oder dass man zur Herstellung von Nitrilen der Formel I (worin $X^1$, $X^2$, $X^3$ und/oder $X^4$ CN bedeutet) die entsprechenden Chlor- oder Bromverbindungen mit einem Cyanid umsetzt.

10. Verwendung der Verbindungen der Formel I

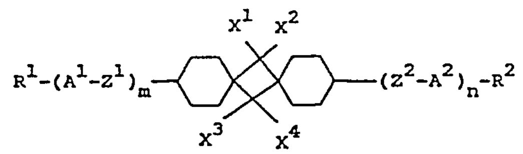
(I)

worin

$R^1$ und $R^2$ jeweils unabhängig voneinander Alkyl mit 1 bis 12 C-Atomen, worin auch eine oder mehrere nicht benachbarte $CH_2$-Gruppen durch $-O-$, $-CO-$, $-CO-O-$, $-O-CO$, $-O-COO-$ und/oder $-CH=CH-$ (trans) ersetzt sein können, einer der Reste $R^1$ und $R^2$ auch H, F, Cl, Br, J, CN, $NO_2$, NCS,

$A^1$ und $A^2$ jeweils unabhängig voneinander trans-1,4-Cyclohexylen, worin auch eine oder zwei nicht benachbarte $CH_2$-Gruppen durch $-O-$ und/oder $-S-$ ersetzt sein können oder 1,4-Phenylen, worin auch eine oder mehrere CH-Gruppen durch N ersetzt sein können, $A^1$ und $A^2$ gegebenenfalls auch lateral bzw. axial substituiert sein können durch F, Cl, CN, $CH_3$,

$Z^1$ und $Z^2$ jeweils unabhängig voneinander $-CO-O-$, $-O-CO$, $-CH_2CH_2-$, $-CH_2O-$, $-OCH_2-$ oder eine Einfachbindung,

m und n jeweils 0, 1 oder 2,

(m + n) 0, 1 oder 2,

$X^1$, $X^2$, $X^3$

und $X^4$ jeweils unbhängig voneinander H, F, Cl oder CN,

bedeuten, und eine oder beide der Gruppen $CX^1X^2$ und $CX^3X^4$ auch C=O bedeuten können, mit der Massgabe, dass (m + n) 1 oder 2 ist, wenn beide Gruppen $CX^1X^2$ und $CX^3X^4$ C=O bedeuten, als Komponenten flüssigkristalliner Phasen mit einer optischen Anisotropie $\Delta n$ kleiner oder gleich 0,09.

11. Flüssigkristalline Phase mit mindestens zwei flüssigkristallinen Komponenten, dadurch gekennzeichnet, dass mindestens eine Komponente der Phase eine Verbindung der Formel I nach Anspruch 1 ist.

12. Flüssigkristallanzeigeelement, dadurch gekennzeichnet, dass es eine Phase nach Anspruch 11 enthält.

13. Elektrooptisches Anzeigeelement nach Anspruch 12, dadurch gekennzeichnet, dass es als Dielektrium eine Phase nach Anspruch 11 enthält.

## Claims

1. A dispirotetradecane of the formula I

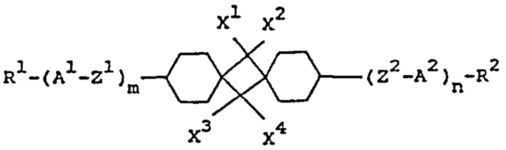

(I)

wherein

$R^1$ and $R^2$ are each independently of the other alkyl containing 1 to 12 carbon atoms wherein one or more non-adjacent $CH_2$ groups may also be replaced by $-O-$, $-CO-$, $-CO-O-$, $-O-CO-$, $-O-COO-$ and/or $-CH=CH-$ (trans), one of the radicals $R^1$ and $R^2$ also being H, F, Cl, Br, I, CN, $NO_2$, NCS,

$A^1$ and $A^2$ are each independently of the other trans-1,4-cyclohexylene wherein one or two non-adjacent $CH_2$ groups may be replaced by $-O-$ and/or $-S-$, or 1,4-phenylene wherein one or more CH groups may also be replaced by N, with it also being possible optionally for $A^1$ and $A^2$ to be substituted laterally or axially by F, Cl, CN, $CH_3$,

$Z^1$ and $Z^2$ are each independently of the other $-CO-O-$, $-O-CO-$, $-CH_2-CH_2-$, $-CH_2O-$, $-OCH_2-$ or a single bond,

m and n are each 0, 1 or 2,

$X^1$, $X^2$, $X^3$

and $X^4$ are each independently of the other H, F, Cl or CN,

and one or both of the groups $CX^1X^2$ and $CX^3X^4$ may also be C=O, with the proviso that (m + n) is 1 or 2 if both groups $CX^1X^2$ and $CX^3X^4$ are C=O.

2. Compounds of formula I according to Claim 1 corresponding to the partial formulae Ia to Id:

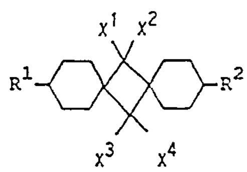

(Ia)

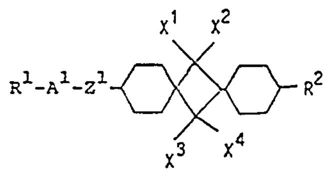

(Ib)

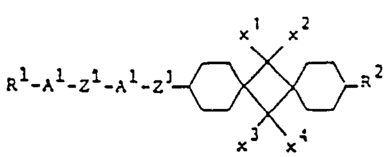

(Ic)

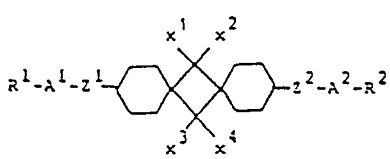

(Id)

wherein $R^1$, $A^1$, $Z^1$, $X^1$, $X^2$, $X^3$, $X^4$, $Z^2$, $A^2$ and $R^2$ have the meaning specified in Claim 1.

3. Compounds according at least to one of the Claims 1-2, characterized in that the structural element

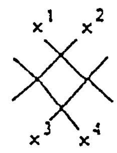

corresponds to one of the formulae 1-15

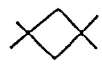
1

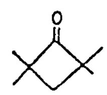
2

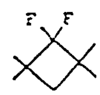
3

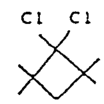
4

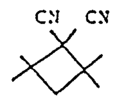
5

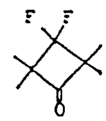
6

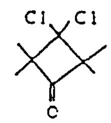
7

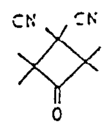
8

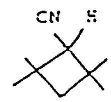
9

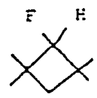
10

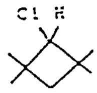
11

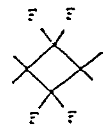
12

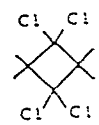
13

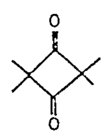
14

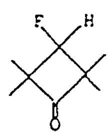
15

4. A compound according at least to one of the Claims 1-3 wherein $A^1$ and $A^2$ are 1,4-cyclohexylene or 1,4-phenylene.

5. A compound according at least to one of the Claims 1-4 wherein $Z^1$ and $Z^2$ are each independently of each other a single bond, $-CO-O-$ or $-O-CO-$.

6. A compound according at least to one of the Claims 1-5 wherein $X^1$, $X^2$, $X^3$ and $X^4$ are each H.

7. A compound according at least to one of the Claims 1-5 wherein one of the groups $CX^1X^2$ and $CX^3X^4$ is C=O.

8. A compound according at least to one of the Claims 1-7 wherein $R^1$ and $R^2$ are each alkyl or alkoxy.

9. A method for the preparation of dispirotetradecanes of the formula I as claimed in Claim 1, wherein corresponding ketenes are dimerized,

or wherein one or both C=O groups in a compound which otherwise corresponds to the formula I but contains two C=O groups, in the 7,14 position is treated with a reducing agent,

or wherein one or both C=groups are converted to the corresponding halogen compounds by reaction with an organic acid halide,

or wherein a compound which otherwise corresponds to the formula 1 but contains one or more reducible groups and/or C-C bonds in place of H atoms is treated with a reducing agent,

or wherein, to produce esters of the formula I (wherein $R^1$ and/or $R^2$ are an alkyl group wherein one or more $CH_2$ groups are replaced by -O-CO-

groups and/or $-CO-O-$groups and/or wherein $Z^1$ and/or $Z^2$ are $-CO-O-$ or $-CO-CO-$), a corresponding carboxylic acid or one of its reactive derivatives is reacted with a corresponding alcohol or one of its reactive derivatives,

or wherein, to produce 1,3-dioxane derivatives or 1,3-dithiane derivatives of the formula I (wherein $A^1$ and/or $A^2$ is 1,3-dioxane-2,5-diyl or 1,3-dithiane-2,5-diyl) a corresponding aldehyde is reacted with a corresponding diol or dithiol,

or wherein to produce ethers of the formula I (wherein $R^1$ and/or $R^2$ is an alkyl group wherein one or more $CH_2$ groups are replaced by O atoms and/or $Z^1$ and/or $Z^2$ is an $-OCH_2-$ or $-CH_2-$ group), a corresponding hydroxyl compound is etherified,

or wherein, to produce nitriles of the formula I (wherein $X^1$, $X^2$, $X^3$ and/or $X^4$ is CN), the corresponding chlorine or bromine compounds are reacted with a cyanide.

10. The use of a compound of the formula I

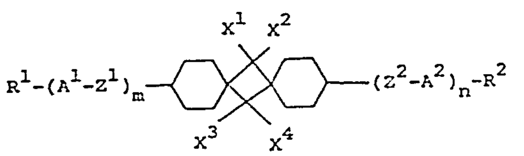 (I)

wherein

$R^1$ and $R^2$ are each independently of the other alkyl containing 1 to 12 carbon atoms wherein one or more non-adjacent $CH_2$ groups may also be replaced by $-O-$, $-CO-$, $-CO-O-$, $-O-CO-$, $-O-COO-$ and/or $-CH=CH-$ (trans), one of the radicals $R^1$ and $R^2$ also being H, F, Cl, Br, I, CN, $NO_2$, NCS,

$A^1$ and $A^2$ are each independently of each other trans-1,4-cyclohexylene wherein one or two non-adjacent $CH_2$ groups may be replaced by $-O-$ and/or $-S-$, or 1,4-phenylene wherein one or more CH groups may also be replaced by N, with it also being possible optionally for $A^1$ and $A^2$ to be substituted laterally or axially by F, Cl, CN, $CH_3$,

$Z^1$ and $Z^2$ are each independently of each other $-CO-O-$, $-O-CO-$, $-CH_2-CH_2-$, $-CH_2O-$, $-OCH_2-$ or a single bond,

m and n are each 0, 1 or 2,

$X^1$, $X^2$, $X^3$

and $X^4$ are each independently of each other H, F, Cl or CN,

and one or both of the groups $CX^1X^2$ and $CX^3X^4$ may also be C=O, with the proviso that (m + n) is 1 or 2 if both groups $CX^1X^2$ and $CX^3X^4$ are C=O, as components of liquid-crystalline phases with an optical anisotropy $\Delta n$ less than or equal to 0.09.

11. A liquid-crystalline phase containing at least two liquid-crystalline components wherein at least one component of the phase is a compound of the formula I according to Claim 1.

12. A liquid-crystalline display element which contains a phase as claimed in Claim 11.

13. An electrooptical display element as claimed in Claim 12, which contains a phase as claimed in Claim 11 as dielectric.

## Revendications

1. Dispirotétradécanes de formule I

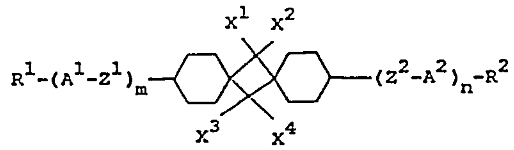 (I)

dans laquelle

$R^1$ et $R^2$ représentent chacun, indépendamment l'un de l'autre, un groupe alkyle en $C_1$-$C_{12}$ dans lequel également un ou plusieurs groupes $CH_2$ non voisins peuvent être remplacés par $-O-$, $-CO-$, $-CO-O-$, $-O-CO-$, $-O-COO-$ et/ou $-CH=CH-$ (trans), l'un des symboles $R^1$ et $R^2$ pouvant également représenter H, F, Cl, Br, I, CN, $NO_2$, NCS,

$A^1$ et $A^2$ représentent chacun, indépendamment l'un de l'autre, un groupe trans-1,4-cyclohexylène dans lequel également un ou deux groupes $CH_2$ non voisins peuvent être remplacés par $-O-$, et/ou $-S-$, ou un groupe 1,4-phénylène dans lequel également un ou plusieurs groupes CH peuvent être remplacés par N, $A^1$ et $A^2$ peuvent également être substitués, en substitution latérale ou axiale par F, Cl, CN, $CH_3$,

$Z^1$ et $Z^2$ représentent chacun, indépendamment l'un de l'autre, $-CO-O-$, $-O-CO-$, $-CH_2CH_2-$, $-CH_2O-$, $-OCH_2-$ ou une liaison simple,

m et n sont chacun égaux à 0, 1 ou 2,

(m + n) est égal à 0, 1 ou 2,

$X^1$, $X^2$, $X^3$

et $X^4$ représentent chacun, indépendamment l'un de l'autre, H, F, Cl ou CN,

un des groupes $CX^1X^2$ et $CX^3X^4$ ou les deux pouvant également consister en groupes C=O, sous réserve que (m + n) est égal à 1 ou 2 lorsque les deux groupes $CX^1X^2$ et $CX^3X^4$ consistent en groupes C=O.

2. Composés de formule I selon la revendication 1, caractérisés en ce qu'ils répondent aux formules partielles Ia à Id

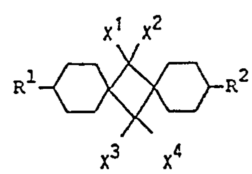 (Ia)

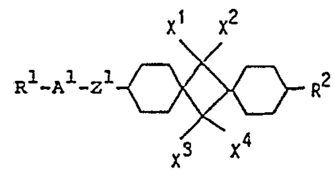 (Ib)

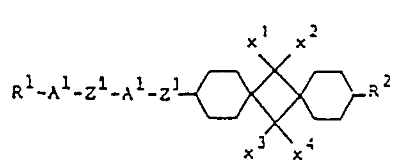 (Ic)

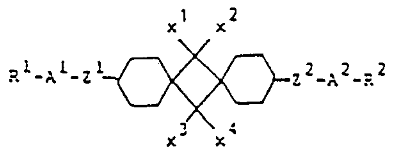 (Id)

dans lesquelles $R^1$, $A^1$, $Z^1$, $X^1$, $X^2$, $X^3$, $X^4$, $Z^2$, $A^2$ et $R^2$ ont les significations indiquées dans la revendication 1.

3. Composés selon l'une des revendications 1 ou 2, caractérisés en ce que l'élément de structure

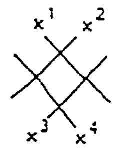

répond à l'une des formules 1 à 15

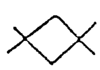
1
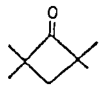
2
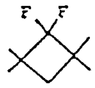
3
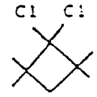
4
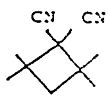
5
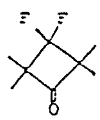
6
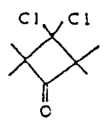
7
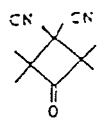
8
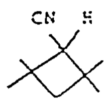
9
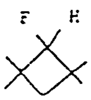
10
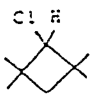
11
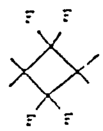
12
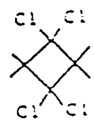
13
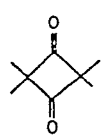
14
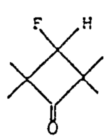
15

4. Composés selon au moins une des revendications 1 à 3, dans lesquels $A^1$ et $A^2$ représentent des groupes 1,4-cyclohexylène ou 1,4-phénylène.

5. Composés selon au moins une des revendications 1 à 4, dans lesquels $Z^1$ et $Z^2$ représentent chacun, indépendamment l'un de l'autre, une liaison simple, un groupe $-CO-O-$ ou $-O-CO-$.

6. Composés selon au moins une des revendications 1 à 5, dans lesquels $X^1 = X^2 = X^3 = X^4 = H$.

7. Composés selon au moins une des revendications 1 à 5, dans lesquels l'un des groupes $CX^1CX^2$ et $CX^3X^4$ consiste en un groupe $C=O$.

8. Composés selon au moins une des revendications 1 à 7, dans lesquels $R^1$ et $R^2$ représentent des groupes alkyle ou alcoxy.

9. Procédé de préparation des dispirotétradécanes de formule I selon la revendication 1, caractérisé en ce que l'on dimérise les cétènes correspondants,

ou bien en ce que, dans un composé répondant par ailleurs à la formule I mais qui porte deux groupes $C=O$ en positions 7,14, on traite un ou deux des groupes $C=O$ par un agent réducteur,

ou bien on convertit un ou deux des groupes $C=O$, par réaction avec un halogénure d'acide organique, en les composés halogénés correspondants,

ou bien on traite par un agent réducteur un composé répondant par ailleurs à la formule I, mais portant à la place d'atomes d'hydrogène un ou plusieurs groupes et/ou liaisons C-C réductibles,

ou bien, pour préparer les esters de formule I (dans lesquels $R^1$ et/ou $R^2$ représentent un groupe alkyle dans lequel un ou plusieurs groupes $CH_2$ sont remplacés par des groupes $-O-CO-$ et/ou $-CO-O-$ et/ou dans lesquels $Z^1$ et/ou $Z^2$ représentent $-CO-O-$ ou $-O-CO-$), on fait réagir un acide carboxylique correspondant ou l'un de ses dérivés réactifs avec un alcool correspondant ou l'un de ses dérivés réactifs,

ou bien, pour préparer des dérivés du 1,3-dioxanne ou du 1,3-dithianne de formule I (dans lesquels $A^1$ et/ou $A^2$ représentent un groupe 1,3-dioxanne-2,5-diyle ou 1,3-dithianne-2,5-diyle), on fait réagir un aldéhyde correspondant avec un diol ou dithiol correspondant respectivement,

ou bien, pour préparer les éthers de formule I (dans lesquels $R^1$ et/ou $R^2$ représentent un groupe alkyle dans lequel un ou plusieurs groupes $CH_2$ sont remplacés par les atomes d'oxygène et/ou $Z^1$ et/ou $Z^2$ représentent un groupe $-OCH_2-$ ou $-CH_2O-$), on éthérifie un composé hydroxylé correspondant,

ou bien, pour préparer les nitriles de formule I (dans lesquels $X^1$, $X^2$, $X^3$ et/ou $X^4$ représentent CN), on fait réagir les dérivés chlorés ou bromés correspondants avec un cyanure.

10. Utilisation des composés de formule I

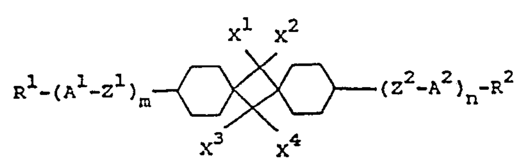 (I)

dans laquelle

$R^1$ et $R^2$ représentent chacun, indépendamment l'un de l'autre, un groupe alkyle en $C_1$-$C_{12}$ dans lequel également un ou plusieurs groupes $CH_2$ non voisins peuvent être remplacés par $-O-$, $-CO-$, $-CO-O-$, $-O-CO-$, $-O-COO-$ et/ou $-CH=CH-$ (trans), l'un des symboles $R^1$ et $R^2$ pouvant également représenter H, F, Cl, Br, I, CN, $NO_2$, NCS,

$A^1$ et $A^2$ représentent chacun, indépendamment l'un de l'autre, un groupe trans-1,4-cyclohexylène dans lequel également un ou deux groupes $CH_2$ non voisins peuvent être remplacés par $-O-$ et/ou $-S-$, ou un groupe 1,4-phénylène dans lequel également un ou plusieurs groupes CH peuvent être remplacés par N, $A^1$ et $A^2$ peuvent également être substitués, en substitution latérale ou axiale, par F, Cl, CN, $CH_3$,

$Z^1$ et $Z^2$ représentent chacun, indépendamment l'un de l'autre, $-CO-O-$, $-O-CO-$, $-CH_2CH_2-$, $-CH_2O-$, $-OCH_2-$ ou une liaison simple,

m et n sont chacun égaux à 0, 1 ou 2,

(m + n) est égal à 0, 1 ou 2,

$X^1$, $X^2$, $X^3$

et $X^4$ représentent chacun, indépendamment les uns des autres, H, F, Cl ou CN,

et l'un des groupes $CX^1X^2$ et $CX^3X^4$ ou les deux peuvent consister en groupes C=O, sous réserve que (m + n) est égal à 1 ou 2 lorsque les deux groupes $CX^1X^2$ et $CX^3X^4$ sont des groupes C=O, en tant que composants de phases à cristaux liquides ayant une anisotropie optique Δn inférieure ou égale à 0,09.

11. Phase à cristaux liquides contenant au moins deux composants à cristaux liquides, caractérisée en ce que l'un au moins des composants de la phase est un composé de formule I selon la revendication 1.

12. Elément d'affichage à cristaux liquides, caractérisé en ce qu'il contient une phase selon la revendication 11.

13. Elément d'affichage électro-optique selon la revendication 12, caractérisé en ce qu'il contient en tant que diélectrique une phase selon la revendication 11.